(19) Europäisches Patentamt
European Patent Office
Office européen des brevets

(11) Veröffentlichungsnummer: **0 312 087 B1**

(12) **EUROPÄISCHE PATENTSCHRIFT**

(45) Veröffentlichungstag der Patentschrift: **15.09.93**

(51) Int. Cl.$^5$: **C07H 15/04**

(21) Anmeldenummer: **88117117.7**

(22) Anmeldetag: **14.10.88**

# (54) Bis-aldonsäureamide und Verfahren zu ihrer Herstellung.

(30) Priorität: **14.10.87 DE 3734853**

(43) Veröffentlichungstag der Anmeldung:
**19.04.89 Patentblatt 89/16**

(45) Bekanntmachung des Hinweises auf die Patenterteilung:
**15.09.93 Patentblatt 93/37**

(84) Benannte Vertragsstaaten:
**AT BE CH DE ES FR GB GR IT LI LU NL SE**

(56) Entgegenhaltungen:
**DE-A- 1 124 938**
**FR-A- 2 587 030**
**US-A- 2 752 334**
**US-A- 3 855 290**

**STÄRKE, Band 33, Nr. 6, 1981, Seiten 202-208, Weinheim, DE; W.N. EMMERLING et al.: "Präparative Methoden zur Herstellung von höheren Maltooligomeren und ihre Verknüpfung mit aliphatischen Diaminen"**

**JOURNAL OF DAIRY SCIENCE, Band 63, Nr. 3, 1980, Seiten 471-473; F. SCHOLNICK et al.: "Iron chelating capacity of gluconamides and lactobionamides"**

**DIE MAKROMOLEKULARE CHEMIE - RAPID COMMUNICATIONS, Band 5, Nr. 7, Juli 1984, Seiten 373-379, Basel, CH; G. ZIEGAST et al.: "Linear and star-shaped hybrid polymers, 2"**

(73) Patentinhaber: **LUITPOLD PHARMA GmbH**
**Postfach 70 12 09**
**D-81312 München(DE)**

(72) Erfinder: **Meinetsberger, Eike, Dr. rer. nat.**
**Friedenheimerstrasse 145**
**D-8000 München 21(DE)**

(74) Vertreter: **Zumstein, Fritz, Dr. et al**
**Dr. F. Zumstein Dipl.-Ing. F. Klingseisen**
**Bräuhausstrasse 4**
**D-80331 München (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.6/3.3.1)

## Beschreibung

Die Erfindung betrifft Bis-aldonsäureamide der allgemeinen Formel I

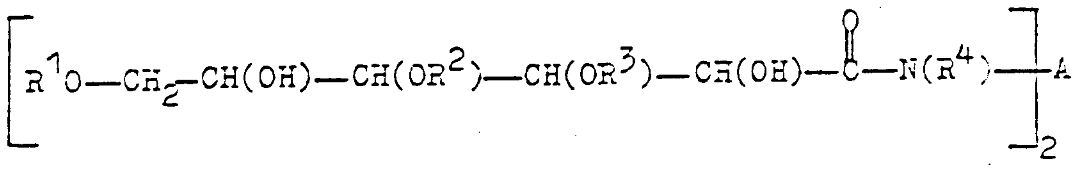

(I)

worin entweder
sämtliche Reste $R^1$, $R^2$ und $R^3$ für ein Wasserstoffatom stehen, oder
zwei der Reste $R^1$, $R^2$ und $R^3$ für ein Wasserstoffatom und der dritte für einen Rest der Formeln II bis VII stehen,

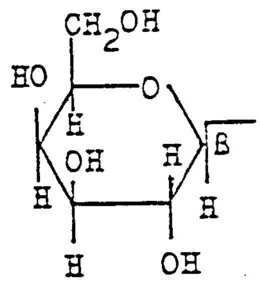

(II)

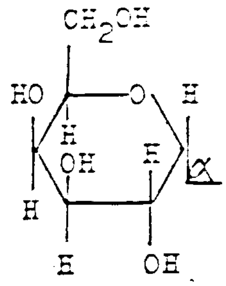

(III)

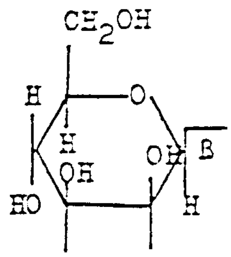

(IV)

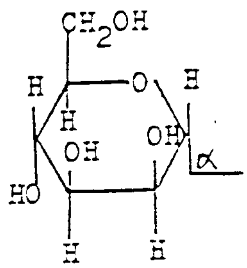

(V)

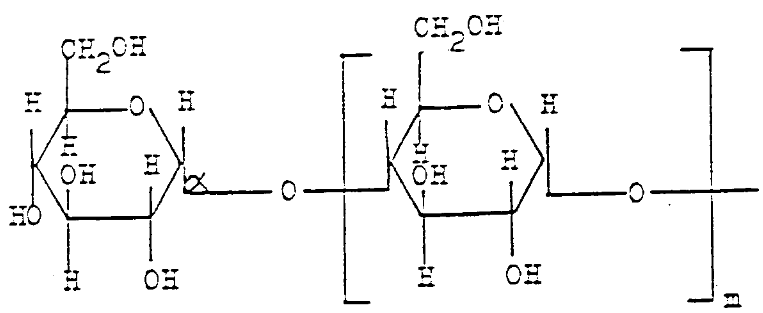

(VI)

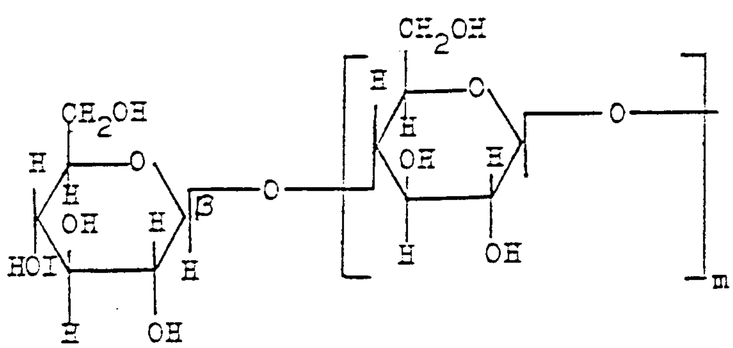

(VII)

m für 0, 1, 2, 3, 4, 5 oder 6 steht,

A in Formel I für einen gegebenenfalls durch einen oder mehrere Reste $-CO_2R^5$ substituierten geradkettigen oder verzweigten, gesättigten Alkylenrest mit 2 mit bis 22 Kohlenstoffatomen steht, und dieser Alkylenrest gegebenenfalls durch bis zu 5 -O-, -S-, -S-S-, $-S(O)_n-$,

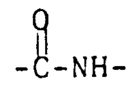

oder/und $-NR^6$-Gruppen oder Cycloalkylen- oder Arylenreste unterbrochen ist, oder A für eine einfache Bindung oder den Rest

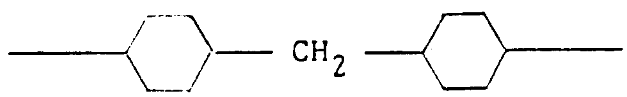

steht,

n 1 oder 2 ist,

$R^4$, $R^5$ und $R^6$ gleichzeitig oder unabhängig voneinander ein Wasserstoffatom oder einen $C_1$-$C_6$-Alkylrest bedeuten,

sowie deren Salze mit anorganischen oder organischen Basen mit den Maßgaben, daß im Fall von Bis-Gluconsäureamiden

a) $R^1$, $R^2$, $R^3$ und $R^4$ nicht gleichzeitig Wasserstoffatome bedeuten und daß,

b) wenn $R^2$ ein Rest der Formel II ist, und gleichzeitig $R^1$, $R^3$ und $R^4$ Wasserstoffatome sind, in diesem Fall A nicht $-(CH_2)_2-$ ist und daß,

c) wenn $R^2$ ein Rest der Formel VI ist, worin m = 0, 1, 2, 3 oder 5, und gleichzeitig $R^1$, $R^3$ und $R^4$ Wasserstoffatome sind und A ein unsubstituierter, geradkettiger Alkylenrest ist, in diesem Fall die Anzahl der Kettenglieder eine ungerade Zahl ist.

Die erfindungsgemäßen Verbindungen stellen wertvolle Zwischenprodukte dar. Man erhält aus ihnen durch Umsetzung mit Sulfatierungsmitteln hochwertige Wirkstoffe mit überraschenden pharmakologischen Eigenschaften. Einige Bis-aldonsäureamide sind bereits bekannt und es wird hierzu auf folgende Literaturstellen verwiesen:

F. Scholnick, P.E. Pfeffer, J. Dairy Sci 63 (3), 471 (1980); W.N. Emmerling, B. Pfannemüller, Starch 33 (6), 202 (1981); G. Ziegast, B. Pfannemüller, Makromol. Chem. 185, 1855 (1984); J. Masse et al., C. R. Acad. Sci., Ser. 3, 301 (1), 27 (1985); K. Dill et al., Inorg. Chim. Acta, 106 (4), 203 (1985).

Die bekannten Verbindungen werden aber in keinem Fall als Zwischenprodukte für die Herstellung der oben genannten Wirkstoffe beschrieben. Emmerling and Pfannemüller verwendeten sie bei enzymatischen Synthesen von Amyloseketten mit Kartoffelphosphorylase. Scholnick und Pfeffer sowie K. Dill et al. untersuchten ihre chelatisierenden Eigenschaften und J. Masse et al. ihren Einfluß auf Wachstum und Chloroyphyllgehalt von Getreide.

Für die im Zusammenhang mit der vorliegenden Erfindung genannten verschiedenen Substituenten beziehungsweise Reste (in den verschiedenen angegebenen Formeln) gelten folgende Erläuterungen:

Die den vorliegenden Bis-aldonsäureamiden zugrundeliegenden Aldonsäuren besitzen die allgemeine Formel X.

$R^1O-CH_2-CH(OH)-CH(OR^2)-CH(OR^3)-CH(OH)-CO_2H$ (X)

worin $R^1$, $R^2$ und $R^3$ die angegebene Bedeutung besitzen. Diese Aldonsäuren können in der D-Form, der L-Form oder in Form ihrer Razemate, bevorzugt in ihrer in der Natur überwiegenden Form vorliegen.

Beispiele für diese Aldonsäuren der Formel X umfassen die Hexonsäuren Allonsäure, Altronsäure, Galaktonsäure, Gluconsäure, Gulonsäure, Idonsäure, Mannonsäure und Talonsäure, bevorzugt Galaktonsäure, Gluconsäure, Gulonsäure und Mannonsäure. Weitere Beispiele sind Derivate dieser Hexonsäuren, welche an den Sauerstoffatomen in 3-, 4- oder 6-Stellung glykosidisch mit einem Rest der Formeln II bis VII verbunden sind. Die Bindung kann hier $\alpha$-oder $\beta$-glykosidisch sein. Bei den Resten II bis V handelt es sich um Galaktopyranosyl-und Mannopyranosylreste. Die Reste VI und VII sind Glucopyranosylreste (für den Fall, daß m = 0) und $\alpha(1\rightarrow4)$-bzw. $\beta(1\rightarrow4)$-verknüpfte Oligogluocopyranosylreste (wenn m = 1 bis 6). Vorzugsweise steht in den Formeln VI und VII der Index m für 0 oder 1. Die mit der Aldonsäure verknüpften Saccharideinheiten liegen normalerweise in der D-Form vor. Beispiele für Hexonsäuren der allgemeinen Formel X, die mit Resten der Formeln II bis VII substituiert sind, sind Glucopyranosylgluconsäuren, Glucopyranosylmannonsäuren, Glucopyranosylgalaktonsäuren, Galaktopyranosylgluconsäuren, Mannopyranosylgluconsäuren, Mannopyranosylmannonsäuren und Oligoglucopyranosylgluconsäuren. Bevorzugt sind hier Lactobionsäure (4-O-β-D-Galaktopyranosylgluconsäure), Gentiobionsäure, Melibionsäure (-D-6-O-α-D-Galaktopyranosylgluconsäure), Mannobionsäure, Cellobionsäure (4-O-β-D-Glucopyranosylgluconsäure), und Maltobionsäure (4-O-α-D-Glucopyranosylgluconsäure) sowie Maltotrionsäure und Cellotrionsäure.

Beispiele für anorganische und organische Salze sind die Ammonium-, Lithium-, Natrium-, Kalium-, Magnesium-, Calcium-, Aluminiumsalze und die Salze mit Ethanolamin, Triethanolamin, Morpholin, Pyridin und Piperidin. Bevorzugt sind die Natrium-, Kalium-, Calcium-, Aluminium- und Ethanolaminsalze.

Beispiele für die Gruppe A darstellende geradkettige oder verzweigte, gesättigte Alkylenreste mit 2 bis 22 Kohlenstoffatomen sind Ethylen-, Tri-, Tetra-, Penta-, Hexa-, Hepta-, Octa-, Nona-, Deca-, Undeca-, Dodeca-, Tetradeca-, Hexadeca-, Octadeca-, Icosa- und Docosamethylen sowie Methylethylen, Methylpropylen, Methylbutylen, Methylpentylen und Dimethylethylen. Bevorzugt sind Ethylen-, Tri-, Tetra-, Hexa-, Nona-, Dodeca- und Docosamethylen sowie Methylethylen und Methylpentylen.

Beispiele für Arylenreste, durch die der Alkylenrest der Gruppe A unterbrochen sein kann, sind Phenylen, Naphthylen, Anthrylen, Phenanthrylen und Fluorenylen. Bevorzugt sind hierbei ortho-, meta- und para-Phenylenreste.

Beispiele für Cycloalkylenreste, durch die der Alkylenrest der Gruppe A unterbrochen sein kann, sind Cyclopentylen, Cyclohexylen, Cycloheptylen und Cyclooctylen, wobei hier 1,3- und 1,4-Cyclohexylen bevorzugt sind.

Vorzugsweise besitzt der geradkettige oder verzweigte, gesättigte Alkylenrest der Gruppe A 2 bis 12 Kohlenstoffatome. Ist der geradkettige oder verzweigte, gesättigte Alkylenrest der Gruppe A durch einen der genannten Reste oder Gruppen unterbrochen, handelt es sich vorzugsweise um 1 oder 2 derartige Reste bzw. Gruppen.

Spezielle Beispiele für definitionsgemäße die Gruppe A darstellende Alkylenreste sind die sich von folgenden α,ω-Diaminen ableitenden Gruppen:

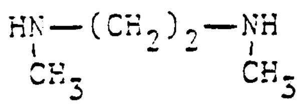

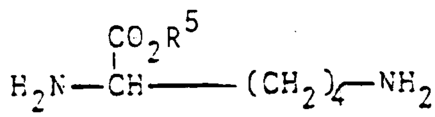

Enantiomere des Lysins ($R^5$ = H) und seiner Ester ($R^5$ = $C_1$-$C_6$-Alkyl)

<u>mit S-Atomen:</u>

$R^5O_2C\text{-}HC(NH_2)\text{-}CH_2S\text{-}CH_2\text{-}(NH_2)CH\text{-}CO_2R^5$

Diastereomere des Lanthionins ($R^5$ = H)
und Ester ($R^5$ = $C_1$-$C_6$-Alkyl)

$R^5O_2C\text{-}HC(NH_2)\text{-}(CH_2)_x\text{-}S\text{-}S\text{-}(CH_2)_x\text{-}(NH_2)CH\text{-}CO_2R^5$

Diastereomere des Cystins (x = 1, $R^5$ = H)
und Ester ($R^5$ = $C_1$-$C_6$-Alkyl)
Diastereomere des Homocystins (x = 2, $R^5$ = H)
und Ester ($R^5$ = $C_1$-$C_6$-Alkyl)

$HO_2C\text{-}CH(NH_2)\text{-}(CH_2)_2\text{-}S\text{-}CH_2\text{-}CH(NH_2)\text{-}CO_2H$

Diastereomere des Cystathionins

mit NH-Gruppen:

| | |
|---|---|
| $H_2N(-CH_2-CH_2-NH)_x-CH_2-CH_2-NH_2$ | x = 1 Diethylentriamin |
| | x = 2 Triethylentetramin |
| | x = 3 Tetraethylenpentamin |
| $H_2N-(CH_2)_2-NH-(CH_2)_3-NH-(CH_2)_2-NH_2$ | 1,9-Diamino-3,7-diazanonan |
| $H_2N-(CH_2)_3-NH-(CH_2)_2-NH-(CH_2)_3-NH_2$ | 1,10-Diamino-4,7-diazadecan |
| $H_2N-(CH_2)_6-NH-(CH_2)_6-NH_2$ | Bis-(6-aminohexyl)amin |
| $H_2N-(CH_2)_3-NH-(CH_2)_4-NH-(CH_3)_3-NH_2$ | Spermin |
| $H_2N-(CH_2)_4-NH-(CH_2)_3-NH_2$ | Spermidin |
| $H_2N-(CH_2)_3-NH-(CH_2)_3-NH-(CH_2)_3-NH_2$ | 1,11-Diamino-4,8-diazaundecan |

mit O-Atomen:

$H_2N-(CH_2)_2-O-(CH_2)_2-NH_2$ Bis-(2-aminoethyl)ether

Vorzugsweise kann die Gruppe A für folgende Reste stehen:

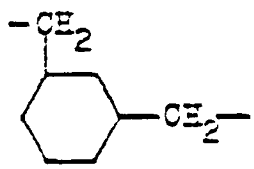 ;

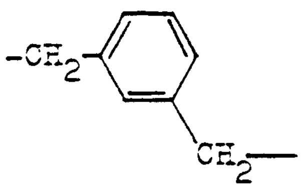 ; 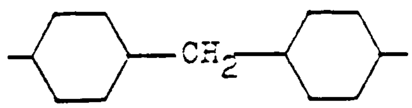 ;

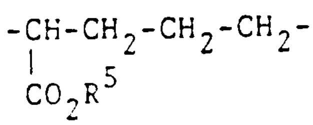

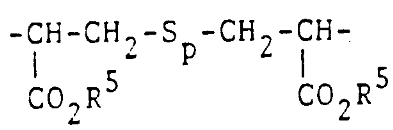 mit p = 1 oder 2

$-(CH_2)_2-S-S-(CH_2)_2-$
$-(CH_2)_3-O-(CH_2)_4-O-(CH_2)_3-$
$-(CH_2)_3-NH-(CH_2)_3-$

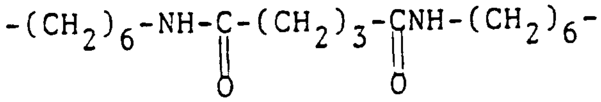

Beispiele für $C_1$ - $C_6$-Alkylreste der Gruppen $R^4$, $R^5$ und $R^6$ sind Methyl, Ethyl, n-Propyl, n-Butyl, n-Pentyl, n-Hexyl, Isopropyl, Isobutyl, tert.-Butyl, Neopentyl, wobei Methyl, Ethyl, n-Propyl, Isopropyl, tert-Butyl und n-Butyl bevorzugt sind.

Die Erfindung betrifft auch Verfahren zur Herstellung der Bis-aldonsäureamide der allgemeinen Formel I. Hierbei läßt man in Analogie zu literaturbekannten Verfahren (s. vorstehende Literaturstellen) die Lactone

der Aldonsäuren X mit einer Diaminoverbindung der allgemeinen Formel $R^4$-HN-A-NHR$^4$, worin $R^4$ die angegebene Bedeutung besitzt, in einem Lösungsmittel reagieren. Die Lactone können dabei sowohl in der 1,5-Lactonform der allgemeinen Formel VIII als auch in der 1,4-Lactonform der allgemeinen Formel IX eingesetzt werden.

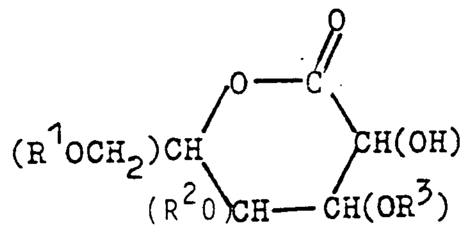

(VIII)

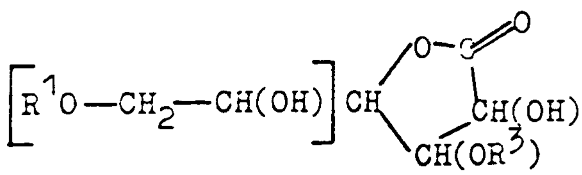

(IX)

Man erhält sie durch Wasserabspaltung aus den Aldonsäuren X. Die Aldonsäuren können nach literaturbekannten Verfahren (z. B.: W. N. Emmerling, B. Pfannemüller, Starch 33 (6), 202 (1981); R. Schaffer, H. S. Isbell, J. Am. Chem. Soc. 81, 2178 (1959), H. W. Diehl et al., Carbohydrate Research 38, 364 (1974)) durch elektrochemische oder Hypohalogenit-Oxidation aus den entsprechenden Aldosen gewonnen werden.

Für die Herstellung der vorliegenden Bis-aldonsäureamide setzt man pro Mol Diaminoverbindung 2 Mol Aldonsäurelacton ein.

Geeignete Lösungsmittel für die Umsetzung sind Methanol, Ethanol, Ethylenglykol, Dimethylsulfoxid, Dimethylformamid oder N-Methylpyrrolidon. Bevorzugt wird Dimethylformamid.

Die Reaktionszeiten betragen mehrere Stunden bis Tage, bevorzugt zwischen 5 und 8 Stunden.

Die Reaktionstemperaturen liegen zwischen Raumtemperatur und den Siedetemperaturen der jeweiligen Lösungsmittel, bevorzugt zwischen 40 °C und 80 °C.

Die Aldonsäureamide kristallisieren entweder aus der Reaktionslösung aus oder man kann sie durch Zusatz eines organischen Lösungsmittels ausfällen. Geeignet hierfür sind Methanol, Ethanol, Isopropanol oder Aceton, vorzugsweise Isopropanol.

In einer bevorzugten Ausführungsform des erfindungsgemäßen Verfahrens werden die Verbindungen der Formel I ohne Isolierung der Lactone aus den Aldonsäuren der Formel X erzeugt.

Hierbei stellt man aus den käuflichen oder nach bekannten Literaturverfahren (siehe oben) synthetisierten Alkali- oder Erdalkalisalzen der Aldonsäuren X mittels eines Kationenaustauschers eine wässrige Lösung der freien Aldonsäuren X her und konzentriert diese weitgehend. Die den Aldonsäuren X entsprechenden Lactone werden nun ohne Isolierung durch Wasserabspaltung erzeugt. Zu diesem Zweck löst man den Rückstand, der ein wasserhaltiges Gemisch aus Aldonsäure und Lacton darstellt, in einem hochsiedenden Lösungsmittel. Beispiele für hochsiedende Lösungsmittel sind Dimethylsulfoxid, Dimethylformamid, N-Methylpyrrolidon, Dimethoxymethylether etc., bevorzugt wird Dimethylformamid. Nun setzt man ein zweites, niedrigsiedendes Lösungsmittel zu, welches ein Azeotrop mit Wasser bilden kann. Geeignete Lösungsmittel sind zum Beispiel n-Pentan, n-Hexan, Cyclohexan, Benzol etc., bevorzugt ist n-Hexan. Am Wasserabscheider wird nun aus den Aldonsäuren quantitativ Wasser abgespalten. Dann destilliert man das niedrigsiedende Lösungsmittel ab und setzt das im zurückbleibenden, hochsiedenden Lösungsmittel befindliche Lacton, ohne Isolierung desselben, mit der Diaminoverbindung um. Die Reaktionstemperaturen liegen dabei zwischen 20 °C und 120 °C, bevorzugt zwischen 50 °C und 80 °C. Die Reaktionsprodukte erhält man durch Ausfällen mit einem organischen Lösungsmittel. Geeignete Lösungsmittel sind beispielsweise Diethyl- und andere Ether, Methanol, Ethanol, Isopropanol, Carbonsäureester und Aceton. Bevorzugt werden Isopropanol und Aceton. Falls erforderlich, können die Verbindungen durch Behandeln mit sauren und basischen Ionenaustauschern von nicht umgesetzten Ausgangsverbindungen befreit werden.

Die Weiterverarbeitung der vorliegenden Bis-aldonsäureamide zu deren polysulfatierten Produkten sowie diese Produkte selber einschließlich ihrer pharmakologischen Eigenschaften werden in der DE-A-3

734 815 der gleichen Anmelderin vom gleichen Anmeldetag; Titel: "Polyschwefelsäureester von Bis-aldonsäureamiden und deren Derivaten, Verfahren zu ihrer Herstellung und Arzneimittel" beschrieben.

Die nachstehenden Beispiele erläutern die Herstellung der vorligenden Verbindungen sowie deren Weiterverarbeitung.

Beispiel 1

N,N'-1,3-Propandiylbis-D-gluconamid

Man löst 7,13 g D(+)-Gluconsäure-1,5-lacton in 40 ml aminfreiem Dimethylformamid und versetzt mit 1,67 ml 1,3-Diaminopropan. Dann erwärmt man auf 60 °C und rührt 5 Stunden. Der entstandene Niederschlag wird abfiltriert, mit Methanol gewaschen und getrocknet. Man erhält 7,96 g eines weißen Pulvers.

| | |
|---|---|
| Schmelzpunkt: | 165 - 173 °C |
| IR (KBr): | ν = 3540, 2960, 2915, 2890, 1660, 1537, 1100, 1040 $cm^{-1}$ |
| $^1$H-NMR ($D_2O$): | δ 1,76 (dt, 2H, 6,5Hz); 3,30 (t, 4H, 6,5Hz); 3,4 - 4,0 (m, 8H); 4,09 (m, 2H); 4,30 (d, 2H, 3Hz); 4,70 ($H_2O$, i.St.) |

Beispiel 2

N,N'-1,12-Dodecandiylbis-D-gluconamid

Man suspendiert 7,1 g D-Gluconsäure-1,5-lacton in 90 ml Dimethylformamid, versetzt mit 4,0 g 1,12-Diaminododecan und rührt das Gemisch 5 Stunden bei 60 °C. Nach Abkühlen rührt man das Gemisch in 0,3 l Methanol ein, sammelt den Feststoff und wäscht ihn mit Methanol. Nun suspendiert man den Feststoff in 1 N HCl, rührt eine Stunde bei Raumtemperatur, sammelt den Feststoff erneut und wäscht ihn mit Wasser, Aceton und schließlich mit Diethylether. Man erhält 9,9 g eines weißen Pulvers.

| | |
|---|---|
| Schmelzpunkt: | 192 - 195 °C |
| IR (KBr): | ν = 2920, 2850, 1630, 1550, 1085, 1027 $cm^{-1}$ |
| $^1$H-NMR (DMSO - $d_6$): | δ 0,7 - 1,8 (m, 20H); 3,06 (m, 4H); 3,25 - 3,75 (m, 8H); 3,75 - 4,2 (m, 4H); 4,40 (S, 10H); 7,51 (t, 2H, 5,5Hz); i. St.: Tetramethylsilan |

Beispiel 3

N,N'-1,3-Propandiylbis [4-O-β-D-galactopyranosyl-D-gluconamid]

Man löst 395,4 g Calciumlactobionat in 1,2 l Wasser und behandelt die Lösung 1 Stunde mit 0,7 l Lewatit S 100 ($H^+$ Form) im Batch-Verfahren. Man saugt ab und wäscht den Austauscher mit 2 x 1 l Wasser. Die vereinigten Eluate engt man im Vakuum weitgehend ein. Nun löst man den glasartigen Rückstand in 800 ml aminfreiem Dimethylformamid, setzt 800 ml n-Hexan zu und erhitzt unter kräftigem Rühren am Wasserabscheider zum Sieden. Nach beendeter Wasserabscheidung destilliert man das n-Hexan ab, versetzt mit 43 ml 1,3-Diaminopropan und rührt 7 Stunden bei 63 °C. Nun rührt man das Gemisch in 5 l Isopropanol ein, sammelt den Feststoff und wäscht mit 1 l Isopropanol. Nach Trocknung erhält man 350 g eines weißen Feststoffes. Zur Reinigung löst man diesen in 2 l Wasser. Die Lösung behandelt man 1 Stunde mit 100 ml Lewatit S 100 ($H^+$-Form), dann mit 100 ml Amberlyst A 21 ($OH^-$-Form). Nach Gefriertrocknung erhält man die Titelverbindung in reiner Form.

| | |
|---|---|
| Schmelzpunkt: | 125 - 132 °C |
| IR (KBr): | ν = 2930, 1645, 1550, 1080 $cm^{-1}$ |
| $^1$H-NMR ($D_2O$): | δ 1,75 (dt, 2H, 6Hz); 3,27 (t, 4H, 6Hz); 3,4 - 4,1 (m, 20H); 4,15 (t, 2H, 3Hz); 4,39 (d, 2H, 3Hz); 4,54 (d, 2H, 7Hz); 4,70 ($H_2O$, i.St.) |
| $^{13}$C-NMR ($D_2O$): | δ 30,79; 38,99; 63,73; 64,65; 71,30; 73,12; 73,74; 74,14; 74,50; 75,06; 75,18; 77,99; 83,71; 106,10; 176,84 i. St.: $CH_3OH$ δ 51,56 |

Beispiel 4

N,N'-1,6-Hexandiylbis[4-O-β-D-galactopyranosyl-D-gluconamid]

Man suspendiert 17,0 g Lactobionsäure-1,5-lacton in 100 ml aminfreiem Dimethylformamid, versetzt mit 2,9 g 1,6-Diaminohexan und rührt 6 Stunden bei 80 °C. Nach Abkühlen wird filtriert, und das Filtrat in 1 l Diethylether eingerührt. Der teilweise ölige Niederschlag wird in 50 ml Wasser gelöst und mit 80 ml Ionenaustauscher (Merck 4765, $H^+$-Form) behandelt. Man filtriert und erhält nach Lyophilisierung 19,5 g farbloses Pulver, das sich ab 175 °C unter Braunfärbung zersetzt.

| | |
|---|---|
| IR: | ν = 2930, 2860, 1645, 1548, 1080 $cm^{-1}$ |
| $^1$H-NMR ($D_2O$): | δ 1,0 - 1,8 (m, 8H); 3,25 (t, 4H, 5,5Hz); 3,3 - 4,1 (m, 20H); 4,15 (t, 2H, 3Hz); 4,38 (d, 2H, 3Hz); 4,55 (d, 2H, 7Hz); 4,70 ($H_2O$)<br>i. St.: 3-Trimethylsilyl-propansulfonsäure-Na-Salz |
| $^{13}$C-NMR ($D_2O$): | δ 28,19; 30,89; 41,63; 63,68; 64,64; 71,25; 73,06; 73,72; 74,12; 74,96; 75,18; 77,97; 83,61; 106,08; 176,46 i. St.: $CH_3OH$ δ 51,54 |

Beispiel 5

N,N'-1,12-Dodecandiylbis[4-O-β-D-galactopyranosyl-D-gluconamid]

Man suspendiert 40,8 g Lactobionsäure-1,5-lacton in 150 ml aminfreiem Dimethylformamid, gibt 12,0 g 1,12-Diaminododecan zu und rührt 6 Stunden bei 60 °C. Unter Rühren tropft man das Gemisch in 1,5 l Isopropanol. Der Niederschlag wird mit Isopropanol gewaschen und in 250 ml Wasser gelöst. Man behandelt die Lösung erst mit 20 ml eines sauren Ionenaustauschers (Lewatit S 100), dann mit einem basischen Ionenaustauscher (Merck 4767). Nach Lyophilisierung erhält man 35,0 g eines farblosen Pulvers.

| | |
|---|---|
| Schmelzpunkt: | 79 - 81 °C |
| IR: | ν = 2920, 2850, 1645, 1550, 1080 $cm^{-1}$ |
| $^1$H-NMR ($D_2O$): | δ 0,8 - 1,9 (m, 20H); 3,24 (t, 4H, 5,5Hz); 3,4 - 4,1 (m, 20H); 4,17 (t, 2H, 3Hz); 4,38 (d, 2H, 3Hz); 4,55 (d, 2H, 7Hz); 4,68 ($H_2O$, i. St.) |
| $^{13}$C-NMR ($D_2O$): | δ 29,02; 31,36; 31,70; 41,86; 63,64; 64,69; 71,20; 73,08; 73,72; 74,17; 75,03; 75,20; 77,97; 83,72; 106,12; 176,24;<br>i. St. $CH_3OH$ δ 51,56 |

Beispiel 6

N,N'-1,9-Nonandiylbis [4-O-β-D-galactopyranosyl-D-gluconamid]

Herstellung und Reinigung analog Beispiel 5. Man erhält aus 15,0 g Lactobionsäure-1,5-lacton und 3,47 g 1,9-Diaminononan 15,0 g der Titelverbindung.

| | |
|---|---|
| IR: | ν = 2930, 2860, 1660, 1545, 1080 $cm^{-1}$ |
| $^1$H-NMR ($D_2O$): | δ 0,9 - 1,8 (m, 14H); 3,20 (t, 4H, 5,5Hz); 3,3 - 4,1 (m, 20H); 4,15 (t, 2H, 3Hz); 4,38 (d, 2H, 3Hz); 4,55 (d, 7Hz); 4,68 ($H_2O$, i.St.) |

Beispiel 7

N,N'-1,12-Dodecandiylbis (4-O-β-D-glucopyranosyl-D-gluconamid)

Man setzt 2,04 g Cellobionsäure-1,5-lacton (H. W. Diehl et al., Carbohydr. Res. 38, 364 (1974)) analog Beispiel 5 mit 0,60 g 1,12-Diaminododecan um und erhält 0,60 g der Titelverbindung.

| | |
|---|---|
| IR: | ν = 2925, 2850, 1645, 1545, 1075, 1040 $cm^{-1}$ |
| $^1$H-NMR ($D_2O$): | δ 0,7 - 1,9 (m, 20H); 3,0 - 4,6 (m, 30H); 4,68 ($H_2O$ i. St.) |

Beispiel 8

N,N'-1,12-Dodecandiylbis (4-O-α-D-glucopyranosyl-D-gluconamid)

Man setzt 20,0 g Calcium-maltobionat (W.N. Emmerling, B. Pfannemüller, Starch 33, 202, (1981)) analog Beispiel 3 mit 1,12-Di-aminododecan um und erhält 17,8 g Produkt.

IR: ν = 2925, 2850, 1650, 1545, 1145, 1075, 1030 $cm^{-1}$
$^1H$-NMR ($D_2O$): δ 0,7 - 1,9 (m, 20H); 3,20 (t, 4H, 5,5Hz); 3,3 - 4,4 (m, 24H); 5,15 (d, 2H, 3Hz); 4,68 ($H_2O$, i.St.)

Beispiel 9

N,N'-1,12-Dodecandiylbis (6-O-α-D-galactopyranosyl)-D-gluconamid)

Man setzt 3,96 g Kalium-melibionat (Sigma-Chemie) analog Beispiel 3 mit 1,00 g 1,12-Diaminododecan um und erhält 3,3 g der Titelverbindung.

Schmelzpunkt: 114 - 123 °C
IR (KBr): ν = 2925, 1855, 1645, 1550, 1150, 1080, 1030, 980 $cm^{-1}$
$^1H$-NMR ($D_2O$): δ 0,8 - 1,8 (m, 20H); 3,20 (m, 4H); 3,4 - 4,2 (m, 22H), 4,29 (d, 2H, 3Hz); 4,95 (s, 2H); 4,68 ($H_2O$, i. St.)

Beispiel 10

N,N'-1,3-Propandiylbis (6-O-α-D-galactopyranosyl-D-gluconamid)

Herstellung analog Beispiel 9. Aus 3,96 g Kalium-melibionat und 0,37 g 1,3-Diaminopropan erhält man 3,0 g Produkt.

Schmelzpunkt: 90 - 96 °C
IR (KBr): ν = 2925, 1645, 1550, 1152, 1080, 1030, 975 $cm^{-1}$
$^1H$-NMR ($D_2O$): δ 1,76 (dt, 2H, 6,5 Hz); 3,30 (t, 4H, 6,5Hz); 3,4 - 4,2 (m, 22H); 4,33 (d, 2H, 3Hz); 4,96 (s, 2H); 4,70 ($H_2O$, i. St.)
$^{13}C$-NMR ($D_2O$): δ 30,73; 38,96; 63,75; 70,94; 74,13; 71,90; 72,12; 73,06; 73,52; 74,52; 75,97; 100,99; 176,91

Beispiel 11

N,N'-α,α'-m-Xyloldiylbis[4-O-β-D-galactopyranosyl-D-gluconamid]

Verwendet man bei einem Verfahren gemäß Beispiel 5 17,0 g Lactobionsäure-1,5-lacton und 3,3 ml 3-(Aminomethyl)-benzylamin, so erhält man auf gleiche Weise 12,2 g der Titelverbindung als farbloses Pulver.

IR: ν = 2920, 1665, 1545, 1080 $cm^{-1}$
$^1H$-NMR ($D_2O$): δ 3,3 - 4,6 (m, 30H); 4,68 ($H_2O$); 7,24 (m, 4H)

Beispiel 12

N,N'-4,4'-Dicyclohexylmethandiylbis[4-O-β-D-galactopyranosyl-D-gluconamid]

Herstellung und Reinigung analog Beispiel 5 aus 17,0 g Lactobionsäure-1,5-lactonund 5,3 g 4,4'-Diamino-dicyclohexylmethan.

Ausbeute: 21,3 g.
IR: ν = 2930, 2850, 1645, 1545, 1080 $cm^{-1}$
$^1H$-NMR ($D_2O$): δ 0,6 - 2,2 (m, 20H); 3,2 - 4,6 (m, 28H), 4,68 ($H_2O$)

Beispiel 13

N,N'-1,6-(3,4-Dithiahexandiylbis)4-O-β-D-galactopyranosyl-D-gluconamid

Man versetzt 17,0 g Lactobionsäure-1,5-lacton und 5,63 g Cystamin Dihydrochlorid in 50 ml aminfreiem DMF bei Raumtemperatur mit 6,9 ml Triethylamin und rührt anschließend 6 Stunden bei 60 °C. Man fällt mit 500 ml Ethanol und behandelt den Niederschlag wie in Beispiel 5 weiter. Man erhält 13,2 g weißes Pulver.

IR: ν = 2925, 1650, 1545, 1080 $cm^{-1}$
$^1H$-NMR ($D_2O$): δ 2,90 (t, 4H, 6Hz); 3,2 - 4,1 (m, 24H); 4,16 (t, 2H, 3Hz); 4,38 (d, 2H, 3Hz); 4,55 (d, 2H, 7 Hz); 4,68 ($H_2O$, i. St.)

Beispiel 14

N,N'-1,7-(4-Azaheptandiylbis)4-O-β-D-galactopyranosyl-D-gluconamid

Man suspendiert 17,0 g Lactobionsäure-1,5-lacton in 100 ml aminfreiem Dimethylformamid, versetzt bei Raumtemperatur mit 2,28 ml Bis-(3-aminopropyl)-amin und rührt 10 Stunden. Dann rührt man 4 Stunden bei 40 °C und filtriert. Das Filtrat rührt man in 900 ml Aceton ein und erhält nach Waschen mit Aceton und Trocknen 23,0 g weiße Kristalle. Diese werden in 80 ml Wasser gelöst und mit 900 ml Aceton gefällt. Der teils ölige Niederschlag wird in 150 ml Wasser gelöst, filtriert und lyophilisiert. Ausbeute: 16,5 g.

IR: v = 2920, 1650, 1545, 1080 $cm^{-1}$

$^1$H-NMR ($D_2O$): δ 1,82 (dt, 4H, 6Hz); 2,91 (t, 4H, 6Hz); 3,30 (t, 4H, 6Hz); 3,45 - 4,6 (m, 26H); 4,68 ($H_2O$)

Beispiel 15

N,N'-1,12-(4,9-Dioxadodecandiylbis)4-O-β-D-galactopyranosyl-D-gluconamid

Herstellung und Reinigung analog Beispiel 5. Man erhält aus 17,0 g Lactobionsäure-1,5-lacton und 5,1 g 1,12-Diamino-4,9-dioxa-dodecan 18,9 g der Titelverbindung.

$^1$H-NMR: δ 1,4 - 2,0 (m, 8H); 3,1 - 4,1 (m, 32H); 4,6 (t, 2H, 3Hz); 4,38 (d, 2H, 3Hz); 4,55 (d, 2H, 7Hz)

Beispiel 16

N,N'-Dimethyl-N,N'-1,2-ethandiylbis (4-O-β-D-galactopyranosyl-D-gluconamid)

Herstellung und Reinigung analog Beispiel 5. Man erhält aus 3,40 g Lactobionsäure-1,5-lacton und 0,44 g N,N'-Dimethylethylendiamin 3,0 g der Titelverbindung.

Schmelzpunkt: 125 - 133 °C

IR (KBr): v = 2930, 1640, 1400, 1075 $cm^{-1}$

$^1$H-NMR ($D_2O$): δ 2,99, 3,16 (2s, 6H); 3,3 - 4,3 (m, 28H); 4,49 (d, 2H, 7Hz); 4,68 ($H_2O$, i. St.)

Beispiel 17

N,N'-1,5-(1-Ethoxycarbonyl)-pentandiylbis (4-O-β-D-galactopyranosyl-D-gluconamid

Man suspendiert 2,47 g Lysinethylester-Dihydrochlorid in 40 ml aminfreiem Dimethylformamid, versetzt mit 3,0 ml Triethylamin und rührt 15 Minuten. Dann gibt man 6,8 g Lactobionsäure-1,5-lacton zu, erwärmt auf 60 °C und rührt 1 Tag. Man filtriert und rührt das Filtrat in 400 ml Isopropanol ein. Der Niederschlag wird gesammelt, in 60 ml Dimethylformamid gelöst und erneut mit 300 ml Isopropanol gefällt. Man wiederholt die Fällung, wäscht mit Isopropanol und Diethylether und erhält so 4,05 g eines weißen Pulvers.

Schmelzpunkt: 106 °C

IR: v = 2930, 1735, 1655, 1550, 1075 $cm^{-1}$

$^1$H-NMR ($D_2O$): δ 1,25 (t, 3H, 7Hz); 1,2 - 2,2 (m, 6H); 3,25 (t, 2H, 5,5Hz); 3,4 - 4,6 (m, 29H); 4,68 ($H_2O$, i. St.)

Beispiel 18

Decanatrium N,N'-1,3-propandiylbis (2,3,4,5,6-penta-O-sulfo-D-gluconamid)

4,30 g N,N'-1,3-Propandiylbis-D-gluconamid werden in 50 ml trockenem Dimethylformamid suspendiert, auf 40 °C erwärmt und unter Rühren mit 23,9 g Pyridin-Schwefeltrioxid-Komplex versetzt. Nach wenigen Minuten fällt das Produkt in Form des Pyridiniumsalzes als Öl aus. Nach 1 Stunde läßt man abkühlen und dekantiert die überstehende Lösung ab. Das Öl wird in 50 ml Wasser gelöst und mit 6 N Natronlauge auf pH = 10 gebracht. Die Lösung wird mit Wasser auf 90 ml aufgefüllt und in 350 ml einer 1 %igen Natriumacetatlösung eingerührt. Der Niederschlag wird mit Methanol gewaschen und getrocknet. Man erhält 18,6 g eines farblosen Pulvers. Dieses löst man in 186 ml Wasser. In die Lösung rührt man 227 ml Methanol ein und läßt 15 Stunden stehen. Man dekantiert vom abgeschiedenen Öl und verreibt dieses mit Methanol. Man wiederholt die Fällung, bis die Titelverbindung rein vorliegt.

Zersetzung ab 190 °C unter Braunfärbung.

IR (KBr): ν = 2960, 1670, 1555, 1250, 1073, 1045, 1019, 770 $cm^{-1}$

$^1$H-NMR ($D_2O$): δ 1,87 (dt, 2H, 7Hz); 3,36 (dt, 4H, 7Hz); 3,9 - 4,6 (m, 4H); 4,8 - 5,4 (m, 8H); 4,68 ($H_2O$, i. St.)

$[\alpha]_{20}^{D}$ = +26,2 (c = 5 in $H_2O$)

| Elementaranalyse: | | |
|---|---|---|
| ber.: | N 22,10 % | S 1,93 % |
| gef.: | N 22,29 % | S 1,83 % |

$^{13}$C-NMR ($D_2O$): δ 29,97; 39,69; 69,06; 77,68; 78,10; 78,39; 79,65; 171,33; i. St.: $CH_3OH$ δ 51,56

Beispiel 19

Decanatrium N,N'-1,12-dodecandiylbis (2,3,4,5,6-penta-D-sulfo-D-gluconamid)

Man setzt 5,60 g N,N'-1,12-Dodecandiylbis-D-gluconamid analog Beispiel 18 mit 25,5 g Pyridin-Schwefeltrioxid-Komplex um und erhält 20,5 g rohes Produkt. Das reine Produkt gewinnt man durch Gelchromatographie einer wässrigen Lösung an einer Sephadex G 25-Säule. Nach Gefriertrocknung erhält man ein farbloses Pulver, das sich zwischen 175 °C und 189 °C unter Braunfärbung zersetzt.

IR (KBr): ν = 2930, 2855, 1665, 1555, 1250, 1072, 1042, 1010 $cm^{-1}$

$^1$H-NMR ($D_2O$): δ 1,0 - 1,9 (m, 20H); 3,32 (m, 4H); 4,2 - 4,6 (m, 4H); 4,9 - 5,3 (m, 8H); 4,68 ($H_2O$, i.St.)

$^{13}$C-NMR ($D_2O$): 28,72; 30,52; 31,02; 42,30; 69,22; 77,67; 78,34; 78,65; 79,91; 171,09; i. St. $CH_3OH$ δ 51,56

Beispiel 20

Hexadecanatrium N,N'-1,3-propandiylbis[2,3,5,6-tetra-O-sulfo-4-O-(2,3,4,6-tetra-O-sulfo-β-D-galactopyranosyl)-D-gluconamid]

Man löst 79,1 g Calciumlactobionat in 240 ml Wasser und behandelt die Lösung mit 240 ml Lewatit S 100 ($H^+$-Form). Man wäscht den Ionenaustauscher mit 3 x 200 ml Wasser und engt die vereinigten Lösungen so weit wie möglich ein. Der glasartige Rückstand wird in 700 ml aminfreiem Dimethylformamid gelöst und mit 600 ml n-Hexan am Wasserabscheider zum Sieden erhitzt. Nach beendeter Wasserabscheidung dampft man das n-Hexan ab und versetzt die Lösung bei Raumtemperatur mit 7,7 g 1,3-Diaminopropan in 50 ml Dimethylformamid. Nach 5 stündigem Rühren bei 60 °C läßt man auf ca. 30 °C abkühlen, verdünnt mit 450 ml Dimethylformamid und gibt rasch portionsweise unter Rühren 400 g Pyridin-Schwefeltrioxid-Komplex zu. Man rührt 1 Stunde zwischen 40 und 45 °C und läßt abkühlen. Man dekantiert vom abgeschiedenen Öl, löst dieses in 500 ml Wasser und stellt die Lösung mit 30 %iger Natronlauge auf pH = 10 ein. Man ergänzt mit Wasser auf ein Volumen von 1,5 l und rührt die Lösung in 4,5 l einer 1 %igen methanolischen Natriumacetatlösung ein. Der Niederschlag wird mit 1 l Methanol verrührt, abgesaugt und getrocknet. Man erhält 250 g eines gelblichen Pulvers. Dieses löst man in 2 l Wasser, versetzt mit 250 ml 30 %igem Hydrogenperoxid und rührt 1 Stunde bei 45 °C. Nach dem Abkühlen neutralisiert man und ergänzt mit Wasser auf 2,5 l. Die Lösung rührt man in 3,06 l Methanol ein und läßt 15 Stunden stehen. Man dekantiert von abgeschiedenem Öl und verreibt dieses mit Methanol. Nach dem Trocknen erhält man 188,5 g farbloses Pulver. Man wiederholt den Fällungsvorgang viermal und erhält schließlich ca. 50 g der reinen Titelverbindung als farbloses Pulver, das sich ab 172 °C unter Zersetzung braun färbt und nicht unter 250 °C schmilzt.

IR (KBr): ν = 2965, 1665, 1552, 1250, 1055, 1020, 927, 820 $cm^{-1}$

$^1$H-NMR ($D_2O$): δ 1,82 (t, 2H, 6,5Hz); 3,35 (t, 4H, 6,5Hz); 3,9 - 4,4 (m, 8H); 4,4 - 4,8 (m, + $H_2O$-Signal bei 4,68 als i. St.); 4,8 - 5,4 (m, 10H)

$^{13}$C-NMR ($D_2O$): δ 30,31; 39,77; 68,36; 68,92; 74,22; 77,49; 77,79; 78,39; 78,76; 80,15; 103,55; 171,76; i. St.: $CH_3OH$ δ 51,56

$[\alpha]_{20}^{D}$ = + 13,3° (c = 5 in $H_2O$)

| Elementaranalyse: | | |
|---|---|---|
| ber.: | N: 1,17 % | S: 21,49 % |
| gef.: | N: 1,16 % | S: 21,61 % |

Beispiel 21

Pentadecanatrium-pentadeca-O-sulfo-N,N'-1,3-propandiylbis (4-O-β-D-galactopyranosyl-D-gluconamid)

Man löst 3,77 g N,N'-1,3-Propandiylbis (4-O-β-D-galactopyranosyl-D-gluconamid) in 60 ml trockenem Dimethylformamid und versetzt bei 40 °C unter Rühren portionsweise mit 13,5 g Pyridin-Schwefeltrioxid-Komplex.Nach 1 Stunde arbeitet man wie in Beispiel 18 auf und erhält 10,3 g gelbliches, sulfathaltiges Rohprodukt. Man löst in 90 ml Wasser, versetzt mit 10 ml 30 %igem Hydrogenperoxid und rührt 1 Stunde bei 45 °C. Nach Abkühlen rührt man 230 ml Methanol ein und läßt 15 Stunden stehen. Man dekantiert vom abgeschiedenen Öl ab, verreibt dieses mit Methanol und erhält 6,72 g sulfatfreies Produkt (mit einem Schwefelgehalt von 20,6 %). Man löst in 67 ml Wasser, rührt 82 ml Methanol ein und läßt 15 Stunden stehen. Man dekantiert vom abgeschiedenen Öl und rührt in den Überstand weitere 74 ml Methanol ein. Nach 15 Stunden isoliert man das Öl und wiederholt damit mehrmals die fraktionierte Fällung wie oben, bis die Titelverbindung rein vorliegt. Man erhält 0,53 g farbloses Pulver, das sich ab 180 °C unter Braunfärbung zersetzt.

IR (KBr): ν = 2960, 1660, 1550, 1250, 1055, 1020, 930, 820 $cm^{-1}$

$^1$H-NMR ($D_2O$): δ 1,87 (t, 2H, 6Hz); 3,42 (t, 4H, 6Hz); 3,9 - 4,5 (m, 8H); 4,5 - 4,85 (m + $H_2O$-Signal bei 4,68 als i. St.); 4,85 - 5,3 (m, 10H)

$^{13}$C-NMR ($D_2O$): δ 30,53; 39,79; 68,46; 69,11; 72,28; 74,36; 74,56; 77,43; 77,88; 78,14; 78,49; 79,03; 79,61; 79,84; 80,43; 103,45; 171,82; 172,61; i. St.: $CH_3OH$ δ 51,56

| Elementaranalyse: | | |
|---|---|---|
| ber.: | N 1,23 % | S 21,04 % |
| gef.: | N 1,21 % | S 20,91 % |

Beispiel 22

Hexadecamorpholinium N,N'-1,3-propandiylbis[2,3,5,6-tetra-O-sulfo-4-O-(2,3,4,6-tetra-O-sulfo-β-D-galactopyranosyl)-D-gluconamid]

Man behandelt eine Lösung von 1,76 g des Natriumsalzes aus Beispiel 20 15 Minuten mit 16 ml Lewatit S-100 ($H^+$-Form), filtriert den Ionenaustauscher ab und versetzt das Filtrat mit 1,03 g Morpholin. Nach Lyophilisierung erhält man 2,40 g gelbliches Pulver.

Zersetzung ab 120 °C und Schwarzfärbung bei 210 °C

IR (KBr): ν = 2950, 2780, 1665, 1563, 1450, 1426, 1250, 1097, 1015, 925, 893, 868, 810 $cm^{-1}$

$^1$H-NMR ($D_2O$): δ 1,82 (dt, 2H; 6,5Hz); 3,15 (m, 64H); 3,35 (m, 4H); 3,90 (m, 64H); 4,0 - 4,4 (m, 8H); 4,4 - 4,8 (m, + $H_2O$-Signal bei 4,70 als i. St.); 4,8 - 5,4 (m, 10H)

Beispiel 23

Hexadecanatrium N,N'-1,6-hexandiylbis[2,3,5,6-tetra-O-sulfo-4-O-(2,3,4,6-tetra-O-sulfo-β-D-galactopyranosyl)-D-gluconamid]

Man setzt 16,3 g N,N'-1,6-Hexandiylbis (4-O-β-D-galactopyranosyl-D-gluconamid) mit 75,0 g Pyridin-Schwefeltrioxid-Komplex analog Beispiel 18 um. Nach der ersten Fällung erhält man 56,9 g eines gelblichen Pulvers, das man wie in Beispiel 20 reinigt. Man gelangt schließlich zu ca. 15 g der reinen Titelverbindung in Form eines farblosen Pulvers, das ab 120 °C sintert.

Zersetzung ab 170 °C unter Braunfärbung.

IR (KBr): ν = 2930, 2860, 1655, 1550, 1250, 1055, 1020, 928, 810 $cm^{-1}$

$^1H$-NMR ($D_2O$): δ 1,1 - 1,9 (m, 8H); 3,37 (m, 4H); 3,9 - 4,5 (m, 8H); 4,5 - 4,85 (m + $H_2O$-Signal bei 4,68 als i. St.); 4,85 - 5,3 (m, 10H)

$^{13}C$-NMR ($D_2O$): δ 28,42; 30,74; 42,17; 68,56; 69,01; 74,39; 77,20; 77,80; 78,37; 78,94; 80,47; 103,21; 171,27; i. St. $CH_3OH$ δ 51,56

$[\alpha]_{20}^{D}$ = + 9,9 (c = 5 in $H_2O$)

Beispiel 24

Hexadecanatrium N,N'-1,9-nonandiylbis[2,3,5,6-tetra-O-sulfo-4-O-(2,3,4,6-tetra-O-sulfo-β-D-galactopyranosyl)-D-gluconamid]

Herstellung und Reinigung analog Beispiel 23. Aus 15,0 g N,N'-1,9-Nonandiylbis(4-O-β-D-galactopyranosyl-D-gluconamid) und 63,0 g Pyridin-Schwefeltrioxid-Komplex erhält man 45,0 g Rohprodukt. Nach Reinigung: 10,5 g farbloses Pulver.

Zersetzung zwischen 192 - 210 °C unter Braunfärbung

IR (KBr): ν = 2935, 2860, 1665, 1555, 1250, 1057, 1020, 925, 815 $cm^{-1}$

$^1H$-NMR ($D_2O$): δ 0,9 - 1,9 (m, 14H); 3,29 (t, 4H, 6,5Hz); 3,8 - 4,45 (m, 8H); 4,45 - 4,8 (m + $H_2O$-Signal bei 4,68 als i. St.); 4,8 - 5,4 (m, 10H)

$^{13}C$-NMR ($D_2O$): δ 28,77; 30,83; 31,09; 31,32; 42,19; 68,69; 68,99; 74,46; 77,12; 77,79; 78,33; 78,93; 80,51; 103,11; 121,21 i. St.: $CH_3OH$, δ 51,56

Beispiel 25

Hexadecanatrium N,N'-1,12-dodecandiylbis[2,3,5,6-tetra-O-sulfo-4-O-(2,3,4,6-tetra-O-sulfo-β-D-galactopyranosyl)-D-gluconamid]

Herstellung und Reinigung analog Beispiel 23. Aus 4,23 g N,N'-1,12-Dodecandiylbis(4-O-β-D-galactopyranosyl-D-gluconamid) und 19,1 g Pyridin-Schwefeltrioxid-Komplex erhält man 13,30 g Rohprodukt. Nach Reinigung: 3,5 g reine Titelverbindung als farbloses Pulver.

Zersetzung zwischen 188 - 198 °C unter Braunfärbung

IR (KBr): ν = 2940, 2880, 1665, 1555, 1250, 1055, 1020, 930, 820 $cm^{-1}$

$^1H$-NMR ($D_2O$): δ 0,9 - 1,9 (m, 20H); 3,35 (t, 4H, 6,5Hz); 3,9 - 4,5 (m, 8H); 4,5 - 4,8 (m, +$H_2O$-Signal bei 4,70 als i. St.); 4,8 - 5,4 (m, 10H)

$^{13}C$-NMR ($D_2O$): δ 28,74; 30,80; 31,09; 31,44; 42,18; 68,76; 68,96; 74,50; 77,08; 77,80; 78,29; 78,94; 80,51; 103,07; 171,19 i. St.: $CH_3OH$ δ 51,56

Beispiel 26

Hexadecanatrium N,N'-1,12-dodecandiylbis[2,3,5,6-tetra-O-sulfo-4-O-(2,3,4,6-tetra-O-sulfo-β-D-glucopyranosyl)-D-gluconamid]

Aus 0,34 g N,N'-1,12-Dodecandiylbis (4-O-β-D-glucopyranosyl-D-gluconamid) und 1,12 g Pyridin-Schwefeltrioxid-Komplex erhält man analog Beispiel 23 0,68 g rohes bzw. 0,10 g reines Produkt.

Zersetzung ab 148 °C bis 159 °C unter Braunfärbung.

IR (KBr): ν = 2930, 2855, 1670, 1560, 1250, 1070, 995, 935, 800 $cm^{-1}$

$^1H$-NMR ($D_2O$): δ 0,8 - 1,8 (m, 20H); 3,30 (m, 4H); 3,7 - 4,8 (m + $H_2O$-Signal bei 4,68 als i. St.); 4,8 - 5,3 (m, 10H)

$^{13}C$-NMR ($D_2O$): δ 28,92; 30,95; 31,32; 31,64; 42,34; 69,20; 70,12; 75,57; 77,44; 77,67; 77,85; 79,33; 79,41; 79,97; 81,08; 102,53; 171,27; i. St.: $CH_3OH$ δ 51,56

Beispiel 27

Hexadecanatrium N,N'-1,12-dodecandiylbis[2,3,5,6-tetra-O-sulfo-4-O-(2,3,4,6-tetra-O-sulfo-α-D-glucopyranosyl)-D-gluconamid]

Aus 12,8 g N,N'-1,12-Dodecandiylbis (4-O-α-D-glucopyranosyl-D-gluconamid) und 64,6 g Pyridin-Schwefeltrioxid-Komplex erhält man analog Beispiel 23 47,5 g rohes bzw. 3,0 g reines Produkt.

Zersetzung von 175 °C bis 189 °C unter Braunfärbung.

IR (KBr): ν = 2930, 2860, 1660, 1560, 1250, 1000, 943, 805 $cm^{-1}$

$^{1}$H-NMR ($D_2O$): δ 1,0 - 1,9 (m, 20H); 3,27 (m, 4H); 4,0 - 4,82 (m + Signal für $H_2O$ bei 4,68 als i. St.); 4,82 - 5,25 (m, 10H); 5,52 (d, 2H, 3Hz)

$^{13}$C-NMR ($D_2O$): 28,84; 30,69; 31,15; 31,47; 42,41; 68,51; 69,29; 71,95; 76,14; 76,82; 77,91; 78,30; 78,44; 79,98; 98,93; 171,27

Beispiel 28

Hexadecanatrium N,N'-1,12-dodecandiylbis[2,3,4,5-tetra-O-sulfo-6-O-(2,3,4,6-tetra-O-sulfo-α-D-galactopyranosyl)-D-gluconamid]

Analog Beispiel 23 erhält man aus 3,30 g N,N'-1,12-Dodecandiylbis (6-O-α-D-galactopyranosyl-D-gluconamid) und 14,9 g Pyridin-Schwefeltrioxid-Komplex 9,7 g rohes bzw. 3,4 g reines Produkt, das ab 57 °C sintert.

Zersetzung ab 182 °C unter Braunfärbung.

IR (KBr): ν = 2930, 2855, 1650, 1555, 1250, 1050, 1027, 830 $cm^{-1}$

$^{1}$H-NMR ($D_2O$): δ 1,0 - 1,9 (m, 20H); 3,25 (m, 4H); 3,9 - 4,4 (m, 8H); 4,4 - 4,8 (m + $H_2O$-Signal bei 4,68 als i. St.); 4,8 - 5,25 (m, 10H); 5,38 (d, 2H, 3Hz)

$^{13}$C-NMR ($D_2O$): δ 28,72; 30,58; 31,03; 31,34; 42,30; 69,14; 69,77; 70,66; 74,51; 74,92; 77,91; 78,21; 78,49; 78,93; 80,75; 99,12; 171,26; i. St.: $CH_3OH$ δ 51,56

Beispiel 29

Hexadecanatrium N,N'-1,3-propandiylbis[2,3,4,5-tetra-O-sulfo-6-O-(2,3,4,6-tetra-O-sulfo-α-D-galactopyranosyl)-D-gluconamid]

Aus 0,34 g N,N'-1,3-Propandiylbis (6-O-α-D-galactopyranosyl-D-gluconamid) und 2,0 g Pyridin-Schwefeltrioxid-Komplex erhält man analog Beispiel 23 0,96 g rohes bzw. 0,50 g reines Produkt.

Zersetzung ab 168 °C unter Braunfärbung

IR (KBr): ν = 1640, 1550, 1250, 1050, 1025, 830 $cm^{-1}$

$^{1}$H-NMR ($D_2O$): δ 1,85 (t, 2H, 6,5Hz); 3,35 (t, 4H, 6,5 Hz); 3,9 - 4,4 (m, 8H); 4,4 - 4,8 (m + $H_2O$-Signal bei 4,68 als i. St.); 4,8 - 5,25 (m, 10H); 5,36 (d, 2H, 3Hz)

$^{13}$C-NMR ($D_2O$): δ 30,13; 39,84; 69,17; 69,86; 70,74; 74,53; 74,97; 78,00; 78,17; 78,37; 79,00; 80,81; 99,18; 171,70; i. St.: $CH_3OH$ δ 51,57

Beispiel 30

Hexadecanatrium N,N'-α,α'-m-xyloldiylbis[2,3,5,6-tetra-O-sulfo-4-O-(2,3,4,6-tetra-O-sulfo-β-D-galactopyranosyl)-D-gluconamid]

Aus 12,0 g N,N'-α,α'-m-Xyloldiylbis(4-O-β-D-galactopyranosyl-D-gluconamid) und 58,8 g Pyridin-Schwefeltrioxid-Komplex erhält man analog Beispiel 23 28,0 g rohes bzw. 5,3 g reines Produkt.

Zersetzung ab 157 °C unter Braunfärbung.

IR (KBr): ν = 2960, 1660, 1550, 1250, 1055, 1020, 930, 815 $cm^{-1}$

$^{1}$H-NMR ($D_2O$): δ 3,9 - 4,85 (m + $H_2O$-Signal bei 4,68 als i. St.), 4,85 - 5,4 (m, 10H); 7,38 (s, 4H)

$^{13}$C-NMR ($D_2O$): δ 45,51; 68,63; 69,15; 74,42; 77,24; 77,67; 77,91; 78,49; 79,08; 80,70; 103,29; 128,15; 128,86; 131,64; 140,80; 171,88; i. St. $CH_3OH$, δ 51,56

Beispiel 31

Hexadecanatrium N,N'-4,4'-dicyclohexylmethandiylbis[2,3,5,6-tetra-O-sulfo-4-O-(2,3,4,6-tetra-O-sulfo-β-D-galactopyranosyl)-D-gluconamid]

Analog Beispiel 23 erhält man aus 25,7 g N,N'-4,4'-Dicyclohexylmethandiylbis (4-O-β-D-galactopyranosyl-D-gluconamid) und 114,7 g Pyridin-Schwefeltrioxid-Komplex 70,7 g rohes bzw. 15,2 g reines Produkt, das ab 120 °C sintert.

Zersetzung ab 180 °C unter Braunfärbung.
IR (KBr): ν = 2930, 2860, 1660, 1550, 1250, 1055, 1020, 928, 815 $cm^{-1}$
$^1$H-NMR ($D_2O$): δ 0,6 - 2,4 (m, 20H); 3,65 (m, 2H); 3,9 - 4,5 (m, 8H); 4,5 - 4,85 (m + $H_2O$-Signal bei 4,68 als i. St.); 4,85 - 4,4 (m, 10H)
$^{13}$C-NMR ($D_2O$): δ 30,18; 30,36; 30,75; 34,09; 44,40; 46,20; 49,45; 52,33; 68,27; 68,75; 74,35; 77,80; 78,41; 78,68; 79,49; 104,09; 170,61; i. St. $CH_3OH$ δ 51,56
$[\alpha]_{20}^{D}$ = + 10,0 (c = 5 in $H_2O$)

Beispiel 32

Hexadecanatrium N,N'-1,6-(3,4-dithiahexandiylbis)[2,3,5,6-tetra-O-sulfo-4-O-(2,3,4,6-tetra-O-sulfo-β-D-galactopyranosyl)-D-gluconamid]

Aus 11,2 g N,N'-1,6-(3,4-Dithiahexandiylbis)4-O-β-D-galactopyranosyl-D-gluconamid) und 53,4 g Pyridin-Schwefeltrioxid-Komplex erhält man analog Beispiel 23 38,0 g rohes und 8,5 g reines Produkt.
Zersetzung ab 163 °C unter Braunfärbung.
IR (KBr): ν = 2965, 1665, 1550, 1250, 1055, 1015, 930, 810 $cm^{-1}$
$1_H$-NMR ($D_2O$): δ 2,96 (t, 4H, 6,5Hz); 3,69 (m, 4H); 4,0 - 4,47 (m, 8H); 4,45 - 4,8 (m + $H_2O$-Signal bei 4,68 als i. St.); 4,8 - 5,3 (m, 10H)
$^{13}$C-NMR ($D_2O$): δ 38,72; 41,06; 68,68; 69,05; 74,48; 77,40; 77,87; 78,46; 80,46; 103,48; 171,86; i. St. $CH_3OH$ δ 51,56

Beispiel 33

Hexadecanatrium N,N'-1,7-(4-azaheptandiylbis)[2,3,5,6-tetra-O-sulfo-4-O-(2,3,4,6-tetra-O-sulfo-β-D-galactopyranosyl)-D-gluconamid]

Aus 11,0 g N,N'-1,7-(4-Azaheptandiylbis)4-O-β-D-galactopyranosyl-D-gluconamid und 50,0 g Pyridin-Schwefeltrioxid-Komplex erhält man analog Beispiel 23 15,4 g rohes und 2,2 g reines Produkt.
Zersetzung ab 165 °C unter Braunfärbung.
IR (KBr): ν = 2960, 2925, 2855, 1650, 1550, 1250, 1055, 1020, 927, 820 $cm^{-1}$
$^1$H-NMR ($D_2O$): δ 2,98 (m, 2H); 3,17 (t, 4H, 7Hz); 3,44 (t, 4H, 6Hz); 3,9 - 4,4 (m, 8H); 4,4 - 4,85 (m + $H_2O$-Signal als i. St. bei 4,68); 4,85 - 5,3 (m, 10H)
$^{13}$C-NMR ($D_2O$): δ 28,11; 39,08; 48,12; 68,65; 69,24; 74,45; 76,94; 77,93; 78,46; 79,09; 80,71; 103,13; 172,06

Beispiel 34

Hexadecanatrium N,N'-1,12-(4,9-dioxadodecandiylbis)[2,3,5,6-tetra-O-sulfo-4-O-(2,3,4,6-tetra-O-sulfo-β-D-galactopyranosyl)-D-gluconamid]

Aus 18,2 g N,N'-1,12-(4,9-dioxadodecandiylbis)4-O-β-D-galactopyranosyl-D-gluconamid und 59,0 g Pyridin-Schwefeltrioxid-Komplex erhält man gemäß Beispiel 23 37,1 g rohes und 9,3 g reines Produkt, das ab 120 °C sintert.
Zersetzung ab 170 °C unter Braunfärbung.
IR (KBr): ν = 2960, 2880, 1665, 1555, 1250, 1055, 1022, 928, 815 $cm^{-1}$
$^1$H-NMR ($D_2O$): δ 1,64 (m, 4H); 1,88 (t, 4H, 6,5Hz); 3,0 - 3,9 (m, 12H); 3,9 - 4,45 (m, 8H); 4,45 - 4,8 (m + $H_2O$-Signal bei 4,68 als i. St.); 4,8 - 5,3 (m, 10H)
$^{13}$C-NMR ($D_2O$): δ 27,82; 30,78; 39,02; 68,64; 69,01; 70,54; 72,96; 74,44; 77,02; 77,79; 78,33; 78,94; 80,52; 103,10; 171,45; i. St.: $CH_3OH$ δ 51,56
$[\alpha]_{20}^{D}$ = + 9,0 (c = 5 in $H_2O$)

Beispiel 35

Hexadecanatrium N,N'-dimethyl-N,N'-1,2-ethandiylbis[2,3,5,6-tetra-O-sulfo-4-O-(2,3,4,6-tetra-O-sulfo-β-D-galactopyranosyl)-D-gluconamid]

Aus 2,50 g N,N'-Dimethyl-N,N'-1,2-ethandiylbis (4-O-β-D-galactopyranosyl-D-gluconamid) und 12,4 g Pyridin-Schwefeltrioxid-Komplex erhält man analog Beispiel 23 8,2 g rohes und 1,2 g reines Produkt.

Zersetzung von 188 - 200 °C unter Braunfärbung.

IR (KBr): ν = 2970, 1650, 1250, 1015, 930, 815 $cm^{-1}$

$^{1}$H-NMR ($D_2O$): δ 3,0 - 4,0 ( m mit s bei 3,35; 10H); 4,0 - 4,7 (m, 14H); 4,70 ($H_2O$, i. St.); 4,9 - 5,4 (m, 10H); 5,54 (d, 2H, 4Hz)

$^{13}$C-NMR ($D_2O$): δ 38,96; 48,28; 68,36; 69,25; 74,17; 75,11; 77,26; 77,73; 78,00; 78,45; 78,76; 79,80; 103,35; 171,25; i. St.: $CH_3OH$, δ 51,56

Beispiel 36

Hexadecanatrium N,N'-1,5-(1-ethoxycarbonyl)-pentandiylbis[2,3,5,6-tetra-O-sulfo-4-O-(2,3,4,6-tetra-O-sulfo-β-D-galactopyranosyl)-D-gluconamid]

Aus 3,6 g N,N'-1,5-(1-Ethoxycarbonyl)-pentandiylbis(4-O-β-D-galactopyranosyl-D-gluconamid) und 15,8 g Pyridin-Schwefeltrioxid-Komplex erhält man gemäß Beispiel 23 8,7 g rohes und 1,2 g reines Produkt, das ab 60 °C sintert.

Zersetzung ab 161 °C unter Braunfärbung.

IR (KBr): ν = 1730, 1650, 1550, 1250, 1055, 1020, 930, 810 $cm^{-1}$

$^{1}$H-NMR ($D_2O$): δ 1,0 - 2,2 (m, 9H, mit t bei 1,31, 7 Hz); 3,30 (m, 2H); 3,9 - 4,8 (m mit $H_2O$-Signal bei 4,68 als i. St.); 4,8 - 5,3 (m, 10H)

$^{13}$C-NMR ($D_2O$): δ 15,94; 29,68; 30,54; 33,17; 41,87; 55,93; 65,19; 68,23; 68,53; 69,04; 74,36; 77,33; 79,81; 78,45; 78,80; 79,61; 80,47; 103,36; 103,99; 171,39; 171,65; 176,12

Beispiel 37

N,N'-1,3-Propandiylbis-D-gulonamid

Man löst 3,56 g D-Gulono-γ-lacton und 0,84 ml 1,3-Diaminopropan in 40 ml Dimethylformamid und rührt 6 Stunden bei 60 °C. Nun rührt man das Gemisch in 200 ml Isopropanol ein und wäscht den Niederschlag mit Isopropanol und Diethylether. Man löst den Feststoff in 20 ml Dimethylformamid und fällt nochmals mit 200 ml Isopropanol. Der Niederschlag wird in Wasser gelöst und gefriergetrocknet. Man erhält 2,2 g eines farblosen Pulvers.

Schmelzpunkt: ν = 2930, 2890, 1645, 1545, 1440, 1080 $cm^{-1}$

$^{1}$H-NMR ($D_2O$): δ 1,74 (dt, 2H, 6,5Hz); 3,27 (t, 4H, 6,5Hz); 3,45 - 4,05 (m, 10H); 4,23 (d, 2H, 6Hz); 4,68 ($H_2O$, i. St.)

$^{13}$C-NMR ($D_2O$): δ 30,65; 39,02; 65,13; 72,64; 74,69; 75,00; 75,12; 176,78; i. St. $CH_3OH$ δ 51,56

Beispiel 38

N,N'-1,2-Propandiylbis-D-galactonamid

Analog Beispiel 37 erhält man aus 7,12 g D-Galactono-γ-lacton und 1,48 g 1,2-Diaminopropan 4,1 g der Titelverbindung als farbloses Pulver.

Schmelzpunkt: 183 - 193 °C unter Zersetzung und Braunfärbung

IR (KBr): ν = 2940, 1656, 1552, 1109, 1055, 1044, 1028, 865 $cm^{-1}$

$^{1}$H-NMR ($D_2O$): δ 1,18 (d, 3H, 6Hz); 3,1 - 4,6 (m, 15H); 4,68 ($H_2O$, i. St.)

$^{13}$C-NMR ($D_2O$): δ 19.64; 19.77; 46.16; 47.87; 48.10; 65.90; 71.93; 72.64; 73.49; 177.75; 178.42; 178.54

Beispiel 39

N,N'-1,4-Butandiylbis-L-mannonamid

Analog Beispiel 18 erhält man aus 3,56 g L-Mannono-$\gamma$-lacton und 0,90 g Putrescin 2,4 g der Titelverbindung als farbloses Pulver.

Zersetzung von 181 - 188° C unter Braunfärbung

IR (KBr): v = 2955, 2925, 2855, 1643, 1555, 1231, 1098, 1043, 1031, 880, 740, 640 $cm^{-1}$

$^{1}H$-NMR ($D_2O$): $\delta$ 1.58 (m, 4H); 3.30 (m, 4H); 3.75 (m, 8H) 4.02 (d, 2H, 7Hz); 4.26 (d, 2H, 7Hz); 4.68 ($H_2O$ i. St.)

$^{13}C$-NMR ($D_2O$): $\delta$ 28.42; 41.38; 65.67; 72.58; 72.76; 73.43; 75.19; 177.09;

Beispiel 40

N,N'-Dilactobionoylhydrazin

Analog Beispiel 37 erhält man aus 6,8 g Lactobionsäure-1,5-lacton und 0,5 ml Hydrazinhydrat 6,1 g Rohprodukt. Säulenchromatographie über Fractogel TSK HW 40S liefert das reine Produkt als farbloses Pulver nach Gefriertrocknung.

Beispiel 41

Decanatrium N,N'-1,3-propandiylbis (2,3,4,5,6-penta-O-sulfo-D-gulonamid)

Analog Beispiel 18 erhält man aus 2,2 g N,N'-1,3-Propandiylbis-D-gulonamid und 12,3 g Pyridin-Schwefeltrioxid-Komplex 9,8 g rohes bzw. 6,4 g reines Produkt als farbloses Pulver. Zersetzung ab 185 °C unter Braunfärbung.

IR (KBr): v = 2960, 1675, 1555, 1250, 1070, 1010, 925, 805 $cm^{-1}$

$^{1}H$-NMR ($D_2O$): $\delta$ 1,85 (m, 2H); 3,34 (m, 4H); 4,52 (d, 4H, 3,5Hz); 5,07 (m, 6H); 5,34 (d, 2H, 3,5 Hz); 4,68 ($H_2O$, i. St.)

$^{13}C$-NMR ($D_2O$): $\delta$ 30.05; 39.62; 68.78; 76.28; 76.41; 77.78; 80.14; 171.15; i. St. $CH_3OH$ $\delta$ 51.55

Beispiel 42

Decanatrium N,N'-1,2-propandiylbis (2,3,4,5,6-penta-O-sulfo-D-galactonamid)

Analog Beispiel 18 erhält man aus 3,3 g N,N'-1,2-Propandiylbis-D-galactonamid und 19,5 g Pyridin-Schwefeltrioxid-Komplex 13,0 g rohes bzw. 9,8 g reines Produkt als farbloses Pulver.

Zersetzung ab 191 °C unter Braunfärbung.

IR (KBr): v = 2970, 1665, 1550, 1250, 1065, 1040, 1007, 900 $cm^{-1}$

$^{1}H$-NMR ($D_2O$): $\delta$ 1,26 (d, 3H, 6,5Hz); 2,9 - 4,3 (m, 3H); 4,3 - 4,6 (m, 4H); 4,68 ($H_2O$, i. St.); 4,8 - 5,3 (m, 8H)

$^{13}C$-NMR ($D_2O$): $\delta$ 19.20; 45.94; 46.15; 47.61; 69.07; 78.42; 78.86; 79.90; 170.84; 171.03; 191.93 i. St. $CH_3OH$ $\delta$ 51.57

Beispiel 43

Decanatrium N,N'-1,4-butandiylbis(2,3,4,5,6-penta-O-sulfo-L-mannonamid)

Analog Beispiel 18 erhält man aus 2,5 g N,N'-1,4-Butandiylbis-L-mannonamid und 14,1 g Pyridin-Schwefeltrioxid-Komplex 10,5 g rohes bzw. 7,2 g reines Produkt als farbloses Pulver.

Zersetzung ab 180° C unter Braunfärbung

IR (KBr): v = 2960, 2930, 2850, 1670, 1555, 1250, 1075, 1010, 925 $cm^{-1}$

$^{1}H$-NMR ($D_2O$): $\delta$ 1.65 (m, 4H); 3.31 (m, 4H); 4.43 (m, 4H); 4.8-5.08 (m, 4H); 5.15 (m, 4H); 4.68 ($H_2O$, i. St.)

$^{13}C$-NMR ($D_2O$): $\delta$ 27.98; 41.62; 69.15; 78.81; 79.36; 79.75; 170.93; i. St. $CH_3OH$ $\delta$ 51.55

Beispiel 44

Hexadecanatrium N,N'-bis[2,3,5,6-tetra-O-sulfo-4-O-(2,3,4,6-tetra-O-sulfo-β-D-galactopyranosyl)-gluconoyl]-hydrazin

Analog Beispiel 18 erhält man aus 6,0 g N,N'-Dilactobionoylhydrazin und 33,7 g Pyridin-Schwefeltrioxid-Komplex 17,5 g rohes bzw. 7,3 g reines Produkt.

Beispiel 45

Man löst bei Zimmertemperatur 5,000 kg Trockensubstanz von Hexadecanatrium N,N'-1,3-propandiylbis[2,3,5,6-tetra-O-sulfo-4-O-(2,3,4,6-tetra-O-sulfo-β-D-galactopyranosyl)-D-gluconamid] unter Rühren in 40 l aqua ad iniectabilia. Nach Einstellen des pH-Wertes der Lösung auf 7,5 mit verdünnter Natronlauge ergänzt man mit aqua ad iniectabilia auf 50,00 l und filtriert durch ein Membranfilter der Porenweite 0,2 µm. Die Lösung wird unter aseptischen Bedingungen in Ampullen zu 1 ml abfiltriert und diese abgeschmolzen.

**Patentansprüche**

**Patentansprüche für folgende Vertragsstaaten : AT, BE, CH, DE, FR, GB, GR, IT, LI, LU, NL, SE**

**1.** Bis-aldonsäureamide der allgemeinen Formel I

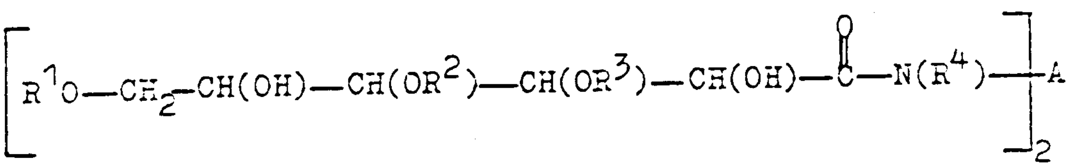

(I)

worin entweder
sämtliche Reste $R^1$, $R^2$ und $R^3$ für ein Wasserstoffatom stehen, oder
zwei der Reste $R^1$, $R^2$ und $R^3$ für ein Wasserstoffatom und der dritte für einen Rest der Formeln II bis VII stehen,

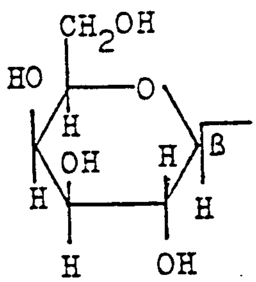

(II)

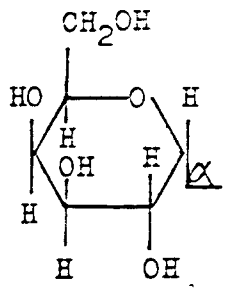

(III)

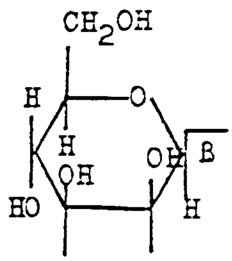

(IV)

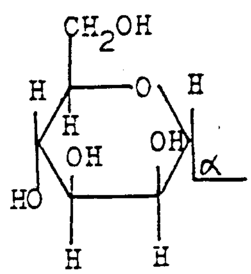

(V)

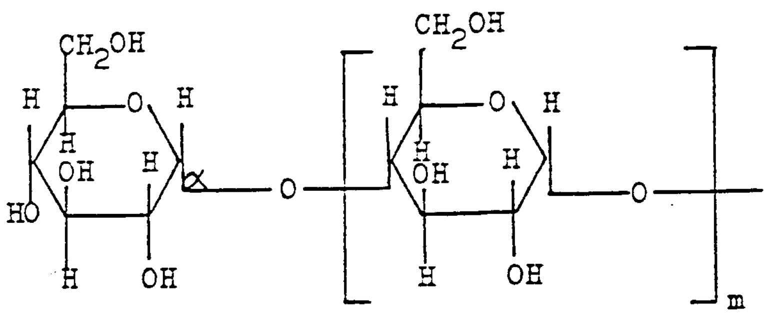

(VI)

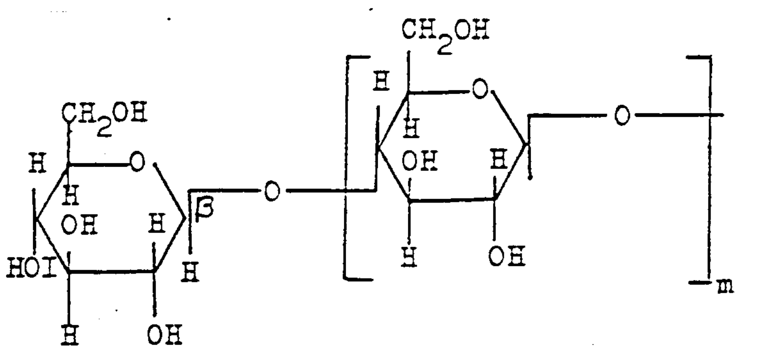

(VII)

m für 0, 1, 2, 3, 4, 5 oder 6 steht,

A in Formel I für einen gegebenenfalls durch einen oder mehrere Reste $-CO_2R^5$ substituierten geradkettigen oder verzweigten, gesättigten Alkylenrest mit 2 bis 22 Kohlenstoffatomen steht, und dieser Alkylenrest gegebenenfalls durch bis zu 5 -O-, -S-, -S-S-, $-S(O)_n-$,

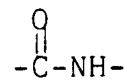

oder/und $-NR^6$-Gruppen oder Cycloalkylen- oder Arylenreste unterbrochen ist, oder A für eine einfache Bindung oder den Rest

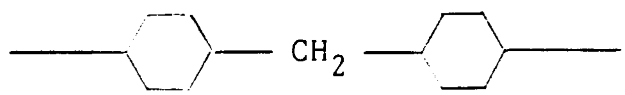

steht,

n 1 oder 2 ist,

$R^4$, $R^5$ und $R^6$ gleichzeitig oder unabhängig voneinander ein Wasserstoffatom oder einen $C_1$-$C_6$-Alkylrest bedeuten, sowie deren Salze mit anorganischen oder organischen Basen mit den Maßgaben, daß im Fall von Bis-Gluconsäureamiden

a) $R^1$, $R^2$, $R^3$ und $R^4$ nicht gleichzeitig Wasserstoffatome bedeuten und daß,

b) wenn $R^2$ ein Rest der Formel II ist, und gleichzeitig $R^1$, $R^3$ und $R^4$ Wasserstoffatome sind, in diesem Fall A nicht $-(CH_2)_2-$ ist und daß,

c) wenn $R^2$ ein Rest der Formel VI ist, worin m = 0, 1, 2, 3 oder 5, und gleichzeitig $R^1$, $R^3$ und $R^4$ Wasserstoffatome sind und A ein unsubstituierter, geradkettiger Alkylenrest ist, in diesem Fall die Anzahl der Kettenglieder eine ungerade Zahl ist.

**2.** Verbindung nach Anspruch 1, dadurch gekennzeichnet, daß $R^2$ und $R^3$ für Wasserstoffatome stehen.

**3.** Verbindungen nach Anspruch 2, dadurch gekennzeichnet, daß $R^1$ den Rest III oder den Rest VI mit m = 0 bedeutet.

**4.** Verbindungen nach Anspruch 1, dadurch gekennzeichnet, daß $R^1$ und $R^3$ für Wasserstoffatome stehen.

**5.** Verbindungen nach Anspruch 4, dadurch gekennzeichnet, daß $R^2$ den Rest II oder den Rest VI mit m = 0 oder den Rest VII mit m = 0 bedeutet.

**6.** Verbindungen nach einem der Ansprüche 2 bis 5, dadurch gekennzeichnet, daß A in Formel I einen Polymethylenrest $-(CH_2)_p-$ mit p = 2 bis 22 bedeutet.

**7.** Verbindungen nach einem der Ansprüche 2 bis 6, dadurch gekennzeichnet, daß A in der Formel I einen Polymethylenrest $-(CH_2)_p-$ mit p = 2 bis 12 bedeutet.

**8.** Verbindungen nach einem der Ansprüche 2 bis 5, dadurch gekennzeichnet, daß A in Formel I einen geradkettigen Alkylenrest mit 2 bis 22 Kohlenstoffatomen darstellt, dessen Kette durch die Gruppen -O-, -S-, -S-S-, $-S(O)_n-$,

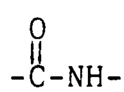

und/oder $-NR^6$- unterbrochen sein kann, wobei n und $R^6$ die in Anspruch 1 angegebenen Bedeutungen besitzen.

**9.** Verbindungen nach einem der Ansprüche 6 bis 8, dadurch gekennzeichnet, daß A in Formel I durch einen, zwei oder mehrere Reste $-CO_2R^5$ substituiert ist, wobei $R^5$ die in Anspruch 1 angegebene Bedeutung hat.

**10.** Verfahren zur Herstellung von Bis-aldonsäureamiden der allgemeinen Formel I gemäß Anspruch 1, dadurch gekennzeichnet, daß man ein Aldonsäurelacton der allgemeinen Formeln VIII oder IX

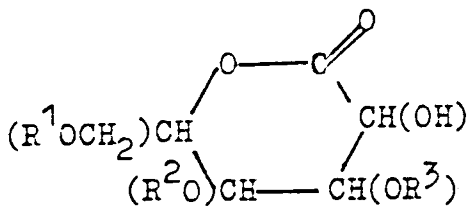 (VIII)

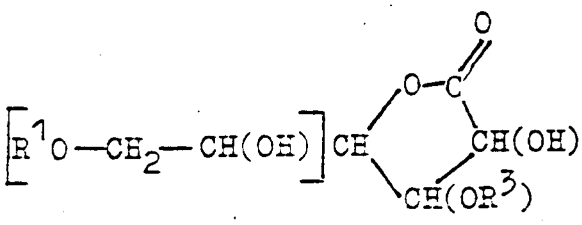 (IX)

mit einer Diaminoverbindung der allgemeinen Formel $R^4HN\text{-}A\text{-}NHR^4$ umsetzt, worin $R^4$ und A die in Anspruch 1 angegebene Bedeutung besitzen.

**11.** Verfahren nach Anspruch 10, dadurch gekennzeichnet, daß man die Aldonsäurelactone der allgemeinen Formeln VIII und IX in situ aus den entsprechenden Aldonsäuren der allgemeinen Formel X

$R^1O\text{-}CH_2\text{-}CH(OH)\text{-}CH(OR^2)\text{-}CH(OR^3)\text{-}CH(OH)\text{-}CO_2H$ (X)

durch Wasserabspaltung herstellt und ohne Isolierung mit der Diaminoverbindung zur Reaktion bringt.

**Patentansprüche für folgenden Vertragsstaat : ES**

**1.** Verfahren zur Herstellung von Bis-aldonsäureamiden der allgemeinen Formel I

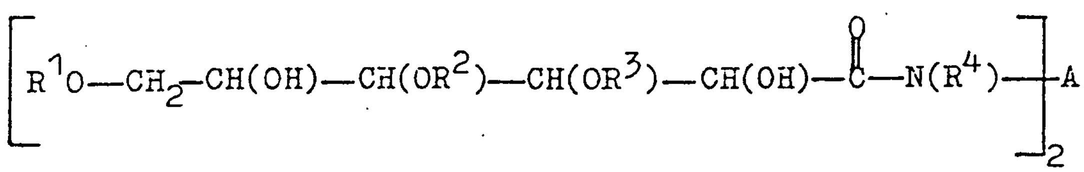 (I)

worin entweder
sämtliche Reste $R^1$, $R^2$ und $R^3$ für ein Wasserstoffatom stehen, oder
zwei der Reste $R^1$, $R^2$ und $R^3$ für ein Wasserstoffatom und der dritte für einen Rest der Formeln II bis VII stehen,

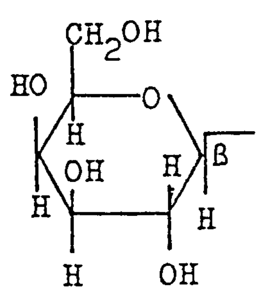 (II)

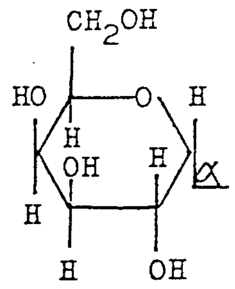

(III)

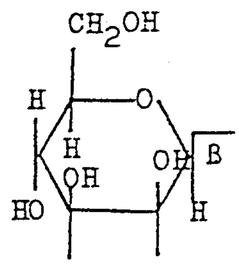

(IV)

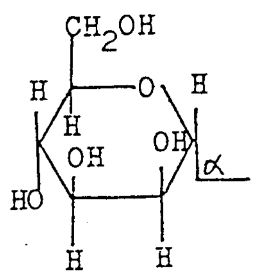

(V)

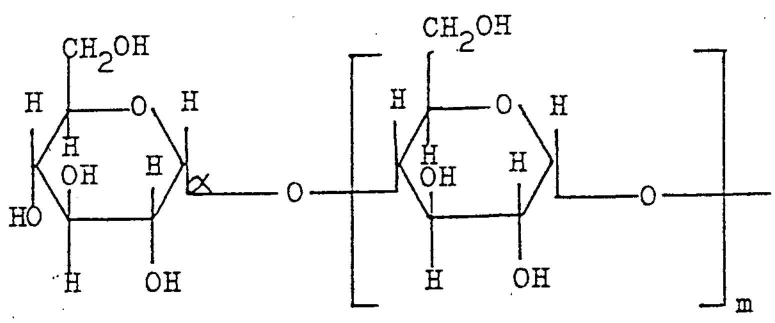

(VI)

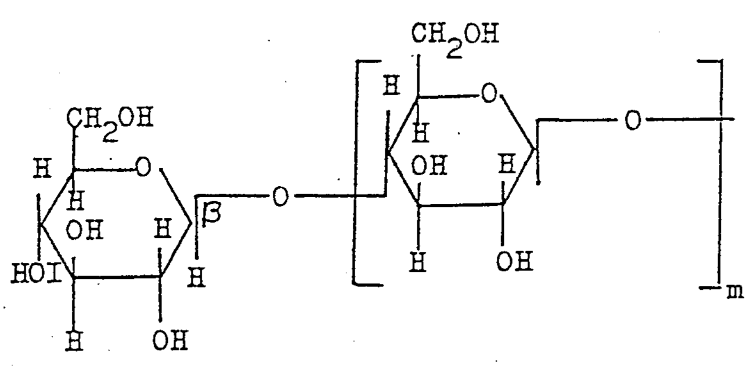

(VII)

m für 0, 1, 2, 3, 4, 5 oder 6 steht,

A in Formel I für einen gegebenenfalls durch einen oder mehrere Reste $-CO_2R^5$ substituierten geradkettigen oder verzweigten, gesättigten Alkylenrest mit 2 bis 22 Kohlenstoffatomen steht, und dieser Alkylenrest gegebenenfalls durch bis zu 5 -O-, -S-, -S-S-, $-S(O)_n-$,

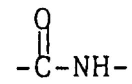

oder/und $-NR^6$-Gruppen oder Cycloalkylen- oder Arylenreste unterbrochen ist, oder A für eine einfache Bindung oder den Rest

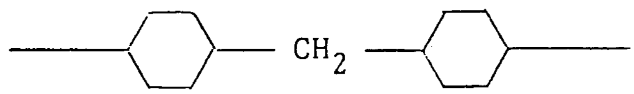

steht,

n 1 oder 2 ist,

$R^4$, $R^5$ und $R^6$ gleichzeitig oder unabhängig voneinander ein Wasserstoffatom oder einen $C_1$-$C_6$-Alkylrest bedeuten, sowie von deren Salzen mit anorganischen oder organischen Basen mit den Maßgaben, daß im Fall von Bis-Gluconsäureamiden

a) $R^1$, $R^2$, $R^3$ und $R^4$ nicht gleichzeitig Wasserstoffatome bedeuten und daß,

b) wenn $R^2$ ein Rest der Formel II ist, und gleichzeitig $R^1$, $R^3$ und $R^4$ Wasserstoffatome sind, in diesem Fall A nicht $-(CH_2)_2-$ ist und daß,

c) wenn $R^2$ ein Rest der Formel VI ist, worin m = 0, 1, 2, 3 oder 5, und gleichzeitig $R^1$, $R^3$ und $R^4$ Wasserstoffatome sind und A ein unsubstituierter, geradkettiger Alkylenrest ist, in diesem Fall die Anzahl der Kettenglieder eine ungerade Zahl ist, dadurch gekennzeichnet, daß man ein Aldonsäurelacton der allgemeinen Formeln VIII oder IX

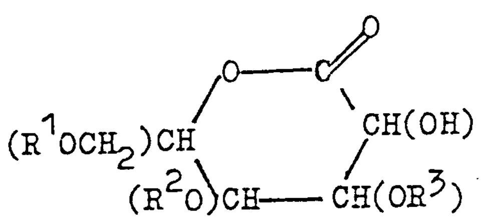

(VIII)

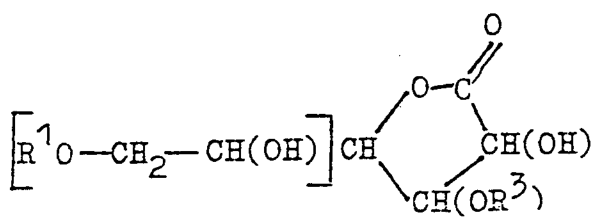

(IX)

mit einer Diaminoverbindung der allgemeinen Formel $R^4HN-A-NHR^4$ umsetzt, worin $R^4$ und A die vorstehend angegebene Bedeutung besitzen.

2. Verfahren nach Anspruch 1 , dadurch gekennzeichnet, daß man die Aldonsäurelactone der allgemeinen Formeln VIII und IX in situ aus den entsprechenden Aldonsäuren der allgemeinen Formel X

$$R^1O\text{-}CH_2\text{-}CH(OH)\text{-}CH(OR^2)\text{-}CH(OR^3)\text{-}CH(OH)\text{-}CO_2H \qquad (X)$$

durch Wasserabspaltung herstellt und ohne Isolierung mit der Diaminoverbindung zur Reaktion bringt.

3. Verfahren nach Anspruch 1 oder 2, dadurch gekennzeichnet, daß $R^2$ und $R^3$ für Wasserstoffatome stehen.

4. Verfahren nach Anspruch 3 , dadurch gekennzeichnet, daß $R^1$ den Rest III oder den Rest VI mit m = 0 bedeutet.

**5.** Verfahren nach Anspruch 1 oder 2, dadurch gekennzeichnet, daß $R^1$ und $R^3$ für Wasserstoffatome stehen.

**6.** Verfahren nach Anspruch 5, dadurch gekennzeichnet, daß $R^2$ den Rest II oder den Rest VI mit m = 0 oder den Rest VII mit m = 0 bedeutet.

**7.** Verfahren nach einem der Ansprüche 3 bis 6 , dadurch gekennzeichnet, daß A in Formel I einen Polymethylenrest $-(CH_2)_p-$ mit p = 2 bis 22 bedeutet.

**8.** Verfahren nach einem der Ansprüche 3 bis 7 , dadurch gekennzeichnet, daß A in der Formel I einen Polymethylenrest $-(CH_2)_p-$ mit p = 2 bis 12 bedeutet.

**9.** Verfahren nach einem der Ansprüche 3 bis 6 , dadurch gekennzeichnet, daß A in Formel I einen geradkettigen Alkylenrest mit 2 bis 22 Kohlenstoffatomen darstellt, dessen Kette durch die Gruppen -O-, -S-, -S-S-, $-S(O)_n-$,

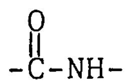

und/oder $-NR^6-$ unterbrochen sein kann, wobei n und $R^6$ die in Anspruch 1 angegebenen Bedeutungen besitzen.

**10.** Verfahren nach einem der Ansprüche 7 bis 9 , dadurch gekennzeichnet, daß A in Formel I durch einen, zwei oder mehrere Reste $-CO_2R^5$ substituiert ist, wobei $R^5$ die in Anspruch 1 angegebene Bedeutung hat.

**Claims**
**Claims for the following Contracting States : AT, BE, CH, DE, FR, GB, GR, IT, LI, LU, NL, SE**

**1.** Bis-aldonic acid amides of the general formula I

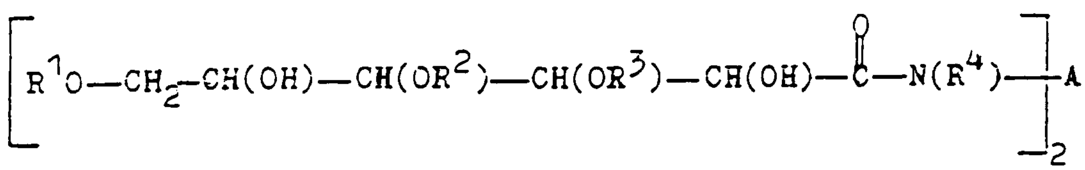
(1)

wherein either
all the radicals $R^1$, $R^2$ and $R^3$ represent a hydrogen atom or
two of the radicals $R^1$, $R^2$ and $R^3$ represent a hydrogen atom and the third represents a radical of the formulae II to VII

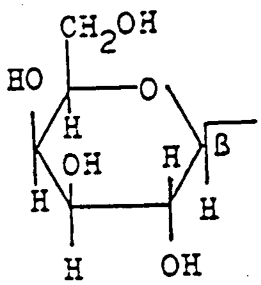

(II)

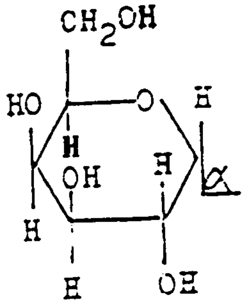

(III)

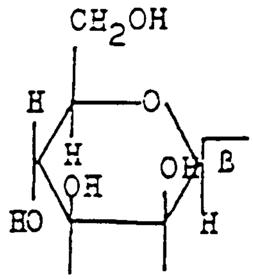

(IV)

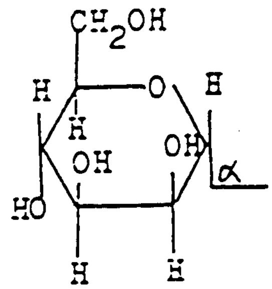

(V)

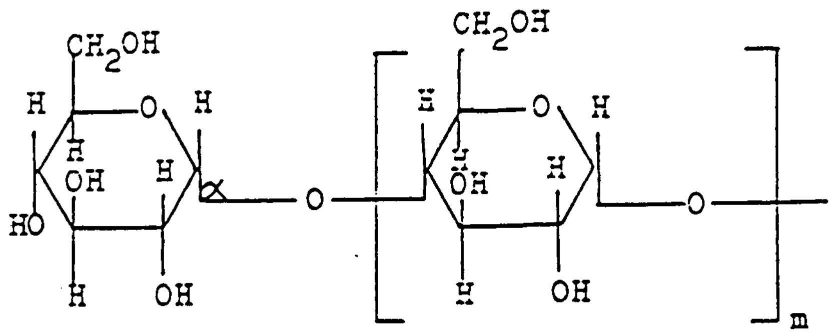

(VI)

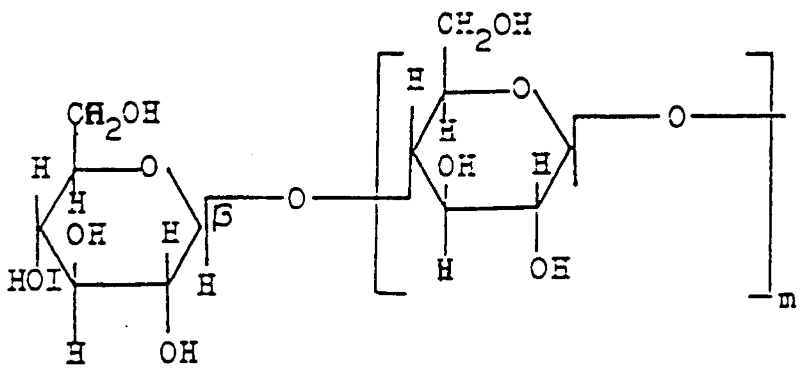

(VII)

m represents 0, 1, 2, 3, 4, 5 or 6,

A in formula I represents a straight-chain or branched, saturated alkylene radical which has 2 to 22 carbon atoms and is optionally substituted by one or more radicals $-CO_2R^5$, and this alkylene radical is optionally interrupted by up to 5 -O-, -S-, -S-S-, $-S(O)_n-$,

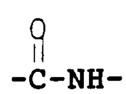

or/and $-NR^6-$ groups or cycloalkylene or arylene radicals, or A represents a single bond or the radical

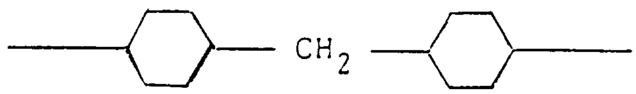

n is 1 or 2 and

$R^4$, $R^5$ and $R^6$ at the same time or independently of one another denote a hydrogen atom or a $C_1$-$C_6$-alkyl radical,

and salts thereof with inorganic or organic bases, with the provisos that, in the case of bis-gluconic acid amides,

a) $R^1$, $R^2$, $R^3$ and $R^4$ do not at the same time denote hydrogen atoms, and that

b) if $R^2$ is a radical of the formula II and at the same time $R^1$, $R^3$ and $R^4$ are hydrogen atoms, in this case A is not $-(CH_2)_2-$, and that

c) if $R^2$ is a radical of the formula VI wherein m = 0, 1, 2, 3 or 5 and at the same time $R^1$, $R^3$ and $R^4$ are hydrogen atoms and A is an unsubstituted straight-chain alkylene radical, in this case the number of chain members is an uneven number.

**2.** Compounds according to Claim 1, characterised in that $R^2$ and $R^3$ represent hydrogen atoms.

**3.** Compounds according to Claim 2, characterised in that $R^1$ denotes the radical III or the radical VI where m = 0.

**4.** Compounds according to Claim 1, characterised in that $R^1$ and $R^3$ represent hydrogen atoms.

**5.** Compounds according to Claim 4, characterised in that $R^2$ denotes the radical II or the radical VI where m = 0 or the radical VII where m = 0.

**6.** Compounds according to one of Claims 2 to 5, characterised in that A in formula I denotes a polymethylene radical $-(CH_2)_p-$ where p = 2 to 22.

**7.** Compounds according to one of Claims 2 to 6, characterised in that A in the formula I denotes a polymethylene radical $-(CH_2)_p-$ where p = 2 to 12.

**8.** Compounds according to one of Claims 2 to 5, characterised in that A in formula I represents a straight-chain alkylene radical having 2 to 22 carbon atoms, the chain of which can be interrupted by the groups -O-, -S-, -S-S-, $-S(O)_n-$,

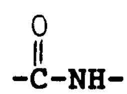

and/or $-NR^6-$, wherein n and $R^6$ have the meanings given in Claim 1.

**9.** Compounds according to one of Claims 6 to 8, characterised in that A in formula I is substituted by one, two or more radicals $-CO_2R^5$, wherein $R^5$ has the meaning given in Claim 1.

**10.** Process for the preparation of bis-aldonic acid amides of the general formula I according to Claim 1, characterised in that an aldonic acid lactone of the general formulae VIII or IX

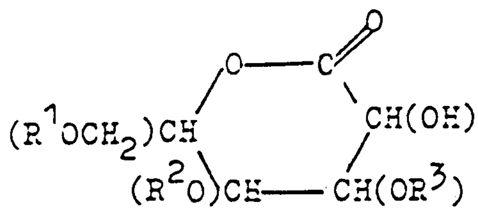

(VIII)

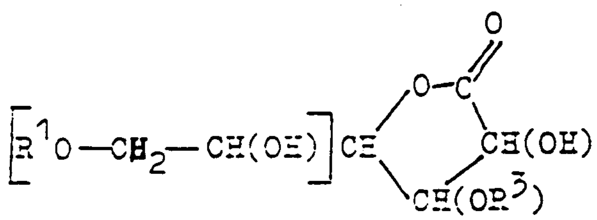

(IX)

is reacted with a diamino compound of the general formula $R^4HN\text{-}A\text{-}NHR^4$, wherein $R^4$ and A have the meaning given in Claim 1.

**11.** Process according to Claim 10, characterised in that the aldonic acid lactones of the general formulae VIII and IX are prepared in situ from the corresponding aldonic acids of the general formula X

$R^1O\text{-}CH_2\text{-}CH(OH)\text{-}CH(OR^2)\text{-}CH(OR^3)\text{-}CH(OH)\text{-}CO_2H$ (X)

by dehydration, and are reacted with the diamino compound without being isolated.

**Claims for the following Contracting State : ES**

**1.** Process for the preparation of bis-aldonic acid amides of the general formula I

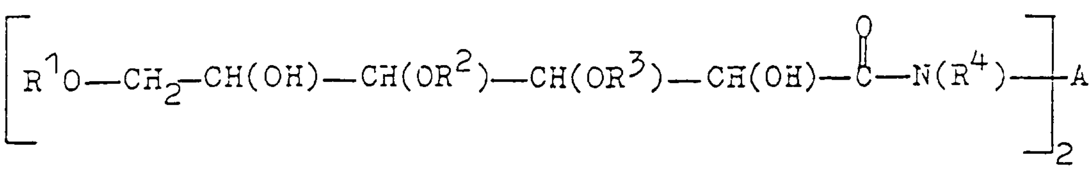

(I)

wherein either
all the radicals $R^1$, $R^2$ and $R^3$ represent a hydrogen atom or
two of the radicals $R^1$, $R^2$ and $R^3$ represent a hydrogen atom and the third represents a radical of the formulae II to VII

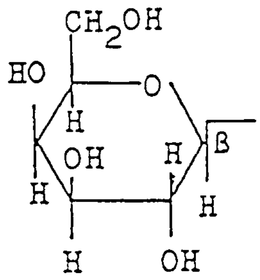

(II)

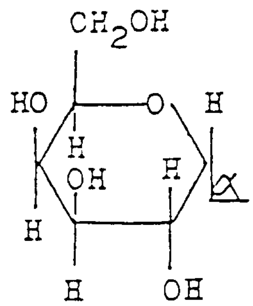

(III)

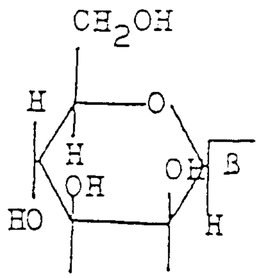

(IV)

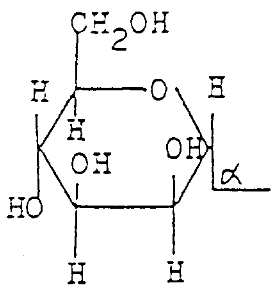

(V)

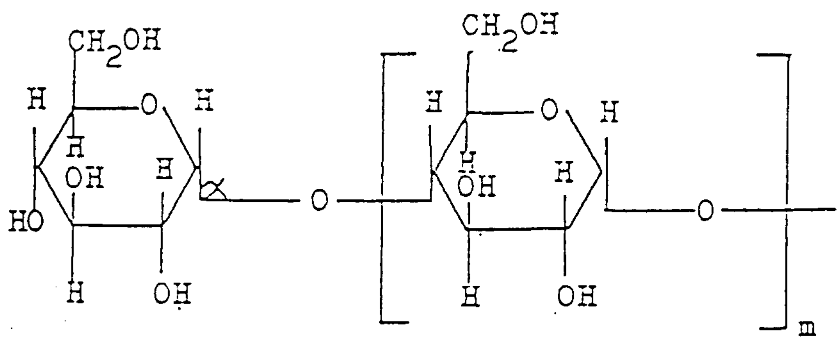

(VI)

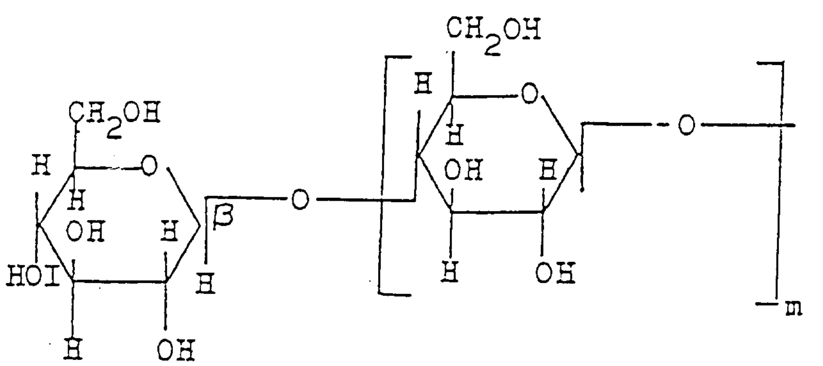

(VII)

m represents 0, 1, 2, 3, 4, 5 or 6,

A in formula I represents a straight-chain or branched, saturated alkylene radical which has 2 to 22 carbon atoms and is optionally substituted by one or more radicals $-CO_2R^5$, and this alkylene radical is optionally interrupted by up to 5 -O-, -S-, -S-S-, $-S(O)_n-$,

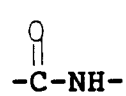

or/and $-NR^6-$ groups or cycloalkylene or arylene radicals, or A represents a single bond or the radical

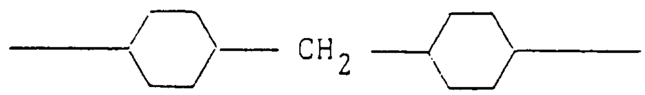

n is 1 or 2 and

$R^4$, $R^5$ and $R^6$ at the same time or independently of one another denote a hydrogen atom or a $C_1$-$C_6$-alkyl radical,

and of salts thereof with inorganic or organic bases, with the provisos that, in the case of bis-gluconic acid amides,

a) $R^1$, $R^2$, $R^3$ and $R^4$ do not at the same time denote hydrogen atoms, and that

b) if $R^2$ is a radical of the formula II and at the same time $R^1$, $R^3$ and $R^4$ are hydrogen atoms, in this case A is not $-(CH_2)_2-$, and that

c) if $R^2$ is a radical of the formula VI wherein m = 0, 1, 2, 3 or 5 and at the same time $R^1$, $R^3$ and $R^4$ are hydrogen atoms and A is an unsubstituted straight-chain alkylene radical, in this case the number of chain members is an uneven number,

characterised in that an aldonic acid lactone of the general formulae VIII or IX

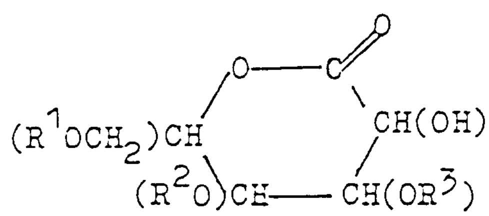

(VIII)

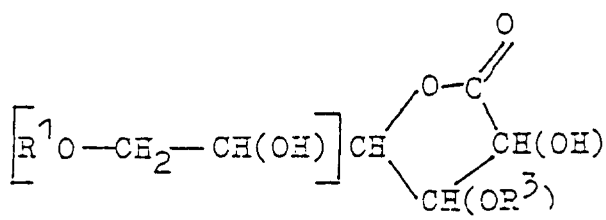

(IX)

is reacted with a diamino compound of the general formula $R^4HN$-A-$NHR^4$, wherein $R^4$ and A have the meaning given above.

**2.** Process according to Claim 1, characterised in that the aldonic acid lactones of the general formulae VIII and IX are prepared in situ from the corresponding aldonic acids of the general formula X

$R^1O$-$CH_2$-CH(OH)-CH($OR^2$)-CH($OR^3$)-CH(OH)-$CO_2H$ (X)

by dehydration, and are reacted with the diamino compound without being isolated.

**3.** Process according to Claim 1 or 2, characterised in that $R^2$ and $R^3$ represent hydrogen atoms.

**4.** Process according to Claim 3, characterised in that $R^1$ denotes the radical III or the radical VI where m = 0.

**5.** Process according to Claim 1 or 2, characterised in that $R^1$ and $R^3$ represent hydrogen atoms.

**6.** Process according to Claim 5, characterised in that $R^2$ denotes the radical II or the radical VI where m = 0 or the radical VII where m = 0.

**7.** Process according to one of Claims 3 to 6, characterised in that A in formula I denotes a polymethylene radical $-(CH_2)_p-$ where p = 2 to 22.

**8.** Process according to one of Claims 3 to 7, characterised in that A in the formula I denotes a polymethylene radical $-(CH_2)_p-$ where p = 2 to 12.

**9.** Process according to one of Claims 3 to 6, characterised in that A in formula I represents a straight-chain alkylene radical having 2 to 22 carbon atoms, the chain of which can be interrupted by the groups -O-, -S-, -S-S-, $-S(O)_n-$,

$$-\overset{\overset{\displaystyle O}{\|}}{C}-NH-$$

and/or $-NR^6-$, wherein n and $R^6$ have the meanings given in Claim 1.

**10.** Process according to one of Claims 7 to 9, characterised in that A in formula I is substituted by one, two or more radicals $-CO_2R^5$, wherein $R^5$ has the meaning given in Claim 1.

**Revendications**
**Revendications pour les Etats contractants suivants : AT, BE, CH, DE, FR, GB, GR, IT, LI, LU, NL, SE**

**1.** Bis-amides d'acides aldoniques de formule générale

$$\left[R^1O-CH_2-CH(OH)-CH(OR^2)-CH(OR^3)-CH(OH)-\overset{\overset{\displaystyle O}{\|}}{C}-N(R^4)\right]_2 A \qquad (I)$$

dans laquelle, soit
les restes $R^1$, $R^2$ et $R^3$ représentent tous un atome d'hydrogène, soit
deux des restes $R^1$, $R^2$ et $R^3$ représentent un atome d'hydrogène et le troisième est un reste d'une des formules II à VII

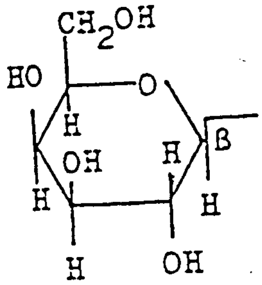

(II)

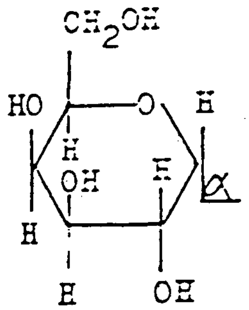

(III)

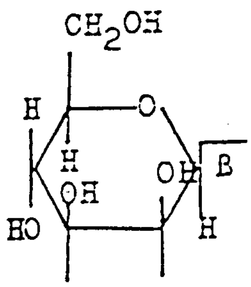

(IV)

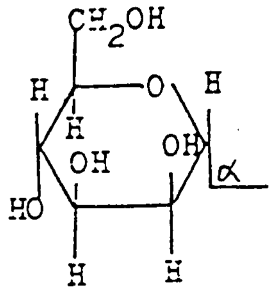

(V)

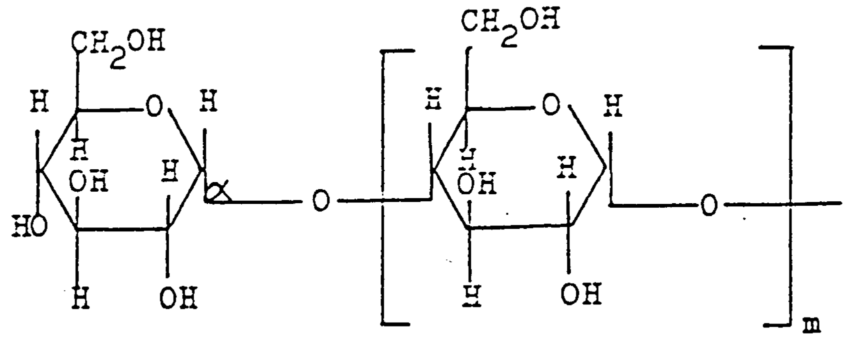

(VI)

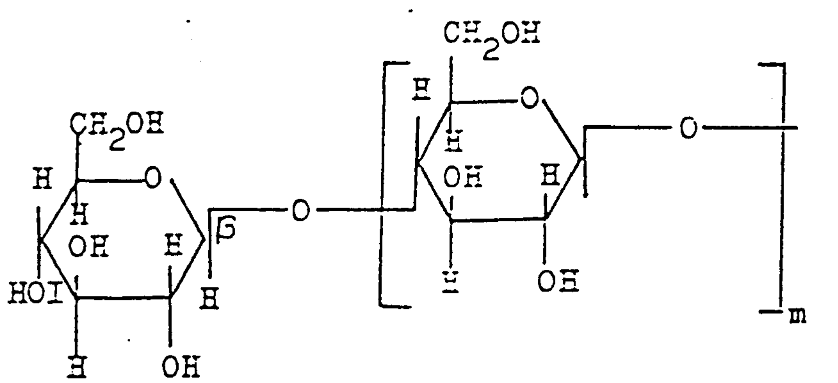

(VII)

m est 0, 1, 2, 3, 4, 5 ou 6,

A dans la formule I représente un reste alkylène de 2 à 22 atomes de carbone saturé, linéaire ou ramifié, éventuellement substitué par un ou plusieurs restes $CO_2R^5$, et ce reste alkylène est éventuellement interrompu jusqu'à 5 fois par des groupes -O-, -S-, -S-S-, $-S(O)_n-$,

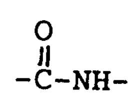

ou/et $-NR^6-$ ou des restes cycloalkylène ou arylène, ou A est une liaison simple ou le reste

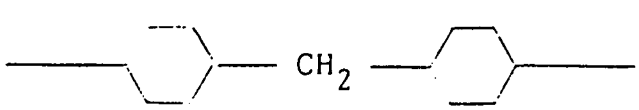

n est 1 ou 2,

$R^4$, $R^5$ et $R^6$ représentent simultanément ou indépendamment l'un de l'autre un atome d'hydrogène ou un reste alkyle en $C_1$-$C_6$,

et leurs sels avec des bases inorganiques ou organiques,

sous réserve que, dans le cas de bis-amides d'acides gluconiques

a) $R^1$, $R^2$, $R^3$ et $R^4$ ne représentent pas simultanément des atomes d'hydrogène et que

b) lorsque $R^2$ est un reste de formule II, et que simultanément $R^1$, $R^3$ et $R^4$ sont des atomes d'hydrogène, A ne soit pas dans ce cas $-(CH_2)_2-$ et que
c) lorsque $R^2$ est un reste de formule VI dans lequel m = 0, 1, 2, 3 ou 5, et que simultanément $R^1$, $R^3$ et $R^4$ sont des atomes d'hydrogène et A un reste alkylène linéaire non substitué,le nombre de chaînons soit dans ce cas un nombre impair.

**2.** Composés selon la revendication 1, caractérisés en ce que $R^2$ et $R^3$ représentent des atomes d'hydrogène.

**3.** Composés selon la revendication 2, caractérisés en ce que $R^1$ représente le reste III ou le reste VI avec m = 0.

**4.** Composés selon la revendication 1, caractérisés en ce que $R^1$ et $R^3$ représentent des atomes d'hydrogène.

**5.** Composés selon la revendication 4, caractérisés en ce que $R^2$ représente le reste II ou le reste VI avec m = 0 ou le reste VII avec m = 0.

**6.** Composés selon l'une des revendications 2 à 5, caractérisés en ce que A, dans la formule I, représente un reste polyméthylène $-(CH_2)_p-$ avec p = 2 à 22.

**7.** Composés selon l'une des revendications 2 à 6, caractérisés en ce que A, dans la formule I, représente un reste polyméthylène $-(CH_2)_p-$ avec p = 2 à 12.

**8.** Composés selon l'une des revendications 2 à 5, caractérisés en ce que A, dans la formule I, représente un reste alkylène linéaire de 2 à 22 atomes de carbone dont la chaîne peut être interrompue par les groupes -O-, -S-, -S-S-, $-S(O)_n-$,

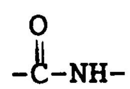

et/ou $-NR^6-$, n et $R^6$ ayant les significations données dans la revendication 1.

**9.** Composés selon l'une des revendications 6 à 8, caractérisés en ce que A, dans la formule I, est substitué par un, deux ou plusieurs restes $-CO_2R^5$, $R^5$ ayant la signification donnée dans la revendication 1.

**10.** Procédé de préparation de bis-amides d'acides aldoniques de formule générale I selon la revendication 1, caractérisé en ce que l'on fait réagir une lactone d'acide aldonique de formule générale VIII ou IX

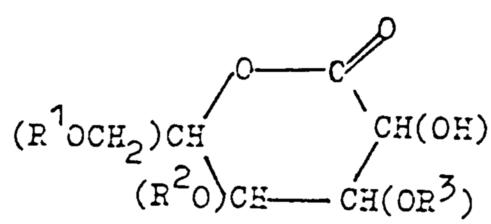

(VIII)

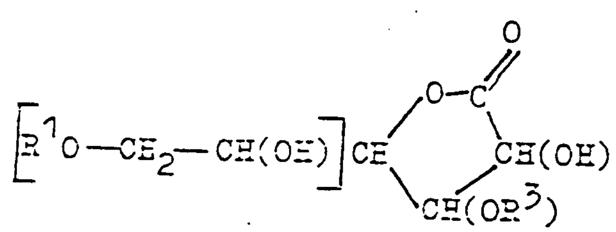

(IX)

avec un composé diamino de formule générale $R^4HN\text{-}A\text{-}NHR^4$, dans laquelle $R^4$ et A ont la signification donnée dans la revendication 1.

**11.** Procédé selon la revendication 10, caractérisé en ce que l'on prépare les lactones d'acides aldoniques de formule générale VIII et IX in situ à partir des acides aldoniques de formule générale X

$$R^1O\text{-}CH_2\text{-}CH(OH)\text{-}CH(OR^2)\text{-}CH(OR^3)\text{-}CH(OH)\text{-}CO_2H \qquad (X)$$

par déshydratation et en ce qu'on les fait réagir, sans les isoler, avec le composé diamino.

**Revendications pour l'Etat contractant suivant : ES**

**1.** Procédé de préparation de bis-amides d'acides aldoniques de formule générale I

$$\left[ R^1O\text{-}CH_2\text{-}CH(OH)\text{-}CH(OR^2)\text{-}CH(OR^3)\text{-}CH(OH)\text{-}\overset{\overset{O}{\|}}{C}\text{-}N(R^4)\right]_2 \text{—}A \qquad (I)$$

dans laquelle, soit
les restes $R^1$, $R^2$ et $R^3$ représentent tous un atome d'hydrogène, soit
deux des restes $R^1$, $R^2$ et $R^3$ représentent un atome d'hydrogène et le troisième est un reste d'une des formules II à VII

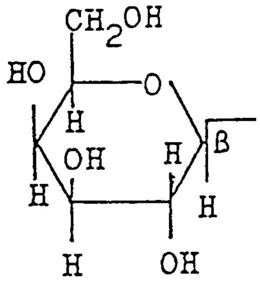

(II)

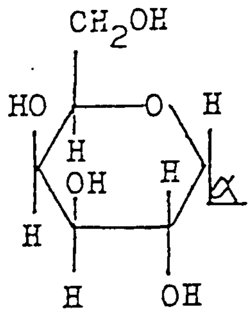

(III)

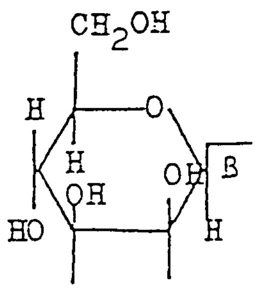

(IV)

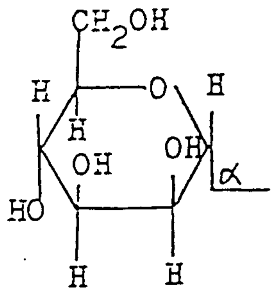

(V)

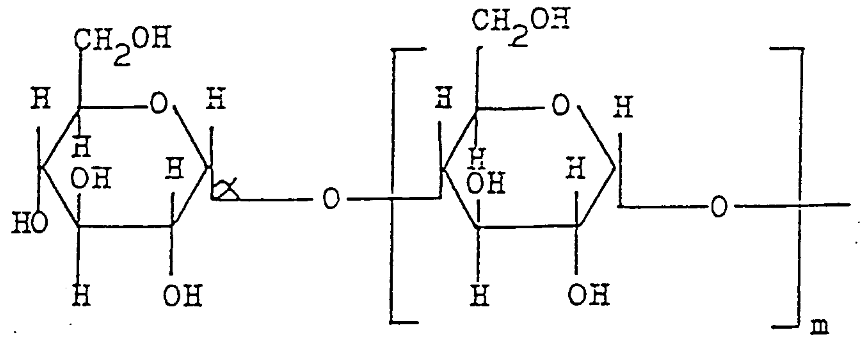

(VI)

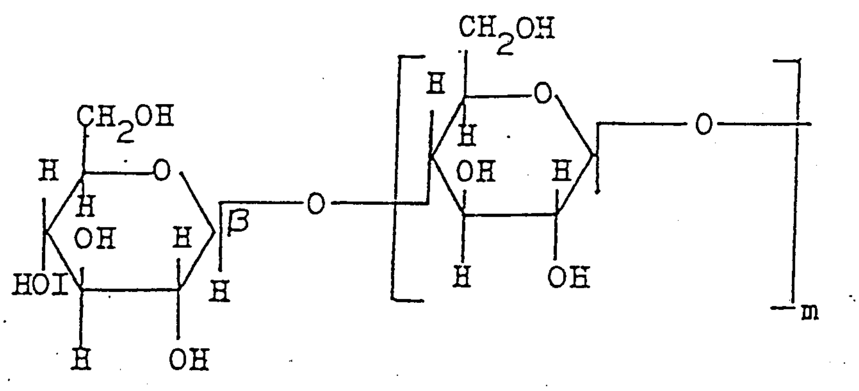

(VII)

m est 0, 1, 2, 3, 4, 5 ou 6,

A dans la formule I représente un reste alkylène de 2 à 22 atomes de carbone saturé, linéaire ou ramifié, éventuellement substitué par un ou plusieurs restes $CO_2R^5$, et ce reste alkylène est éventuellement interrompu jusqu'à 5 fois par des groupes -O-, -S-, -S-S-, $-S(O)_n-$,

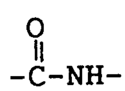

ou/et $-NR^6-$ ou des restes cycloalkylène ou arylène, ou A est une liaison simple ou le reste

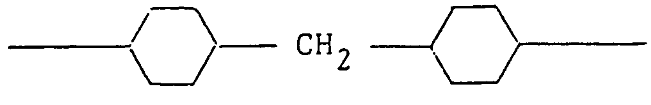

n est 1 ou 2,

$R^4$, $R^5$ et $R^6$ représentent simultanément ou indépendamment l'un de l'autre un atome d'hydrogène ou un reste alkyle en $C_1$-$C_6$,

et leurs sels avec des bases inorganiques ou organiques,

sous réserve que, dans le cas de bis-amides d'acides gluconiques

a) $R^1$, $R^2$, $R^3$ et $R^4$ ne représentent pas simultanément des atomes d'hydrogène et que

b) lorsque $R^2$ est un reste de formule II, et que simultanément $R^1$, $R^3$ et $R^4$ sont des atomes d'hydrogène A ne soit pas dans ce cas $-(CH_2)_2-$ et que

c) lorsque $R^2$ est un reste de formule VI dans lequel m = 0, 1, 2, 3 ou 5, et que simultanément $R^1$, $R^3$ et $R^4$ sont des atomes d'hydrogène et A un reste alkylène linéaire non substitué,le nombre de chaînons soit dans ce cas un nombre impair,

caractérisé en ce que l'on fait réagir une lactone d'acide aldonique de formule générale VIII ou IX

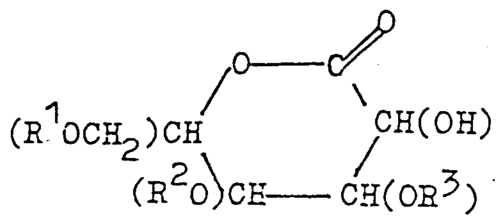

(VIII)

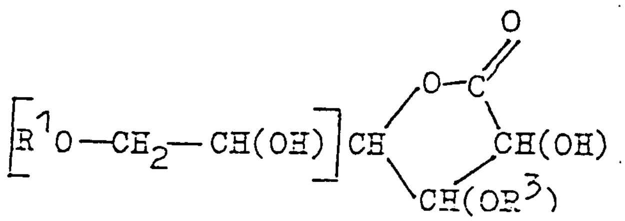

(IX)

avec un composé diamino de formule générale $R^4HN-A-NHR^4$, dans laquelle $R^4$ et A ont la signification donnée ci-dessus.

2. Procédé selon la revendication 1, caractérisé en ce que l'on prépare les lactones d'acides aldoniques de formule générale VIII et IX in situ à partir des acides aldoniques de formule générale X

$R^1O-CH_2-CH(OH)-CH(OR^2)-CH(OR^3)-CH(OH)-CO_2H$ (X)

par déshydratation et en ce qu'on les fait réagir, sans les isoler, avec le composé diamino.

3. Procédé selon la revendication 1 ou 2, caractérisé en ce que $R^2$ et $R^3$ représentent des atomes d'hydrogène.

4. Procédé selon la revendication 3, caractérisé en ce que $R^1$ représente le reste III ou le reste VI avec m = 0.

5. Procédé selon la revendication 1 ou 2, caractérisé en ce que $R^1$ et $R^3$ représentent des atomes d'hydrogène.

6. Procédé selon la revendication 5, caractérisé en ce que $R^2$ représente le reste II ou le reste VI avec m = 0 ou le reste VII avec m = 0.

7. Procédé selon l'une des revendications 3 à 6, caractérisé en ce que A, dans la formule I, représente un reste polyméthylène $-(CH_2)_p-$ avec p = 2 à 22.

8. Procédé selon l'une des revendications 3 à 7, caractérisé en ce que A, dans la formule I, représente un reste polyméthylène $-(CH_2)_p-$ avec p = 2 à 12.

9. Procédé selon l'une des revendications 3 à 6, caractérisé en ce que A, dans la formule I, représente un reste alkylène linéaire de 2 à 22 atomes de carbone dont la chaîne peut être interrompue par les groupes -O-, -S-, -S-S-, $-S(O)_n-$,

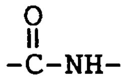

et/ou $-NR^6-$, n et $R^6$ ayant les significations données dans la revendication 1.

**10.** Procédé selon l'une des revendications 7 à 9, caractérisé en ce que A, dans la formule I, est substitué par un, deux ou plusieurs restes $-CO_2R^5$, $R^5$ ayant la signification donnée dans la revendication 1.